# FASCICULE DE BREVET EUROPEEN

(11) **EP 0 796 850 B1**
(45) Date de publication et mention de la délivrance du brevet: **28.07.1999**
(21) Numéro de dépôt: 97400515.9
(22) Date de dépôt: 06.03.1997
(51) Int. Cl.: C07D 231/38, A61K 7/13

(54) **4,5-Diiminopyrazolines, leur procédé de préparation et compositions de teinture les renfermant**
4,5-Diiminopyrazoline, Verfahren zu ihrer Herstellung und diese enthaltende Färbemittel
4,5-Diiminopyrazolines, their process of preparation and dyeing compositions containing them

(30) Priorité: 21.03.1996 FR 9603544
(43) Date de publication de la demande: 24.09.1997
(73) Titulaire: L'OREAL, 75008 Paris (FR)
(72) Inventeur: Malle, Gérard, 77100 Meaux (FR); Vidal, Laurent, 75013 Paris (FR); Samain, Henri, 91570 Bievres (FR)
(74) Mandataire: Miszputen, Laurent

(56) Documents cités:
- WO-A-94/08971
- FR-A- 2 050 990
- CHEMICAL ABSTRACTS, vol. 105, no. 2, 14 Juillet 1986 Columbus, Ohio, US; abstract no. 7925a, L. HENNIG ET AL.: "Azomethine, azo and methine dyes of 5-hydroxy, 5-amino and 5-thiolo-3-methyl-1-phenyl-pyrazole." page 78; colonne 1; XP002021211 & Z. CHEM., vol. 25, no. 11, 1985, pages 402-403,
- Thèse de doctorat de Ute Kern, Technische Hochschule Darmstadt 1988, "Synthese, Reaktionen und Anwendung von Diaminopyrazolen in der Organischen Synthese"
- J. prakt. Chem. (1986), Vol. 328, no. 3, pages 342-348

## Description

La présente invention concerne des 4,5-diiminopyrazolines ou leur forme tautomère 4-ylidèneamino-5-amino-pyrazoles, leur procédé de préparation, leur utilisation à titre de colorant direct dans les applications de teinture des fibres kératiniques, en particulier des fibres kératiniques humaines, et notamment les cheveux, et plus particulièrement les compositions de teinture directe, éclaircissante ou non éclaircissante, les renfermant.

Il est connu de teindre les fibres kératiniques, et en particulier les cheveux, avec des compositions tinctoriales contenant soit des colorants directs, suivant un procédé dit de «coloration directe», soit des précurseurs de colorants d'oxydation suivant un procédé dit de «coloration d'oxydation».
Le procédé de coloration d'oxydation fait intervenir un ou plusieurs précurseurs de colorants d'oxydation, généralement appelés bases d'oxydation (ortho- ou para-phénylènediamines, ortho- ou para- aminophénols) qui sont des composés incolores ou faiblement colorés et qui, sous l'action d'un oxydant, engendrent des composés colorés et colorants par un processus de condensation oxydative. Des coupleurs, encore appelés modificateurs de coloration (métaphénylènediamines, métaaminophénols et métadiphénols), sont le plus souvent associés auxdites bases d'oxydation afin de modifier ou d'enrichir en reflets les colorations «de fond» obtenues par les produits de condensation des bases d'oxydation. Ce procédé de coloration d'oxydation consiste à appliquer sur les fibres kératiniques, des bases ou un mélange de bases et de coupleurs avec de l'eau oxygénée à titre d'agent oxydant, à laisser pauser, puis à rincer les fibres. Les colorations qui en résultent sont permanentes, puissantes, et résistantes aux agents extérieurs, notamment à la lumière, aux intempéries, aux lavages, à la transpiration et aux frottements. Généralement appliqué à pH basique, il permet d'obtenir une teinture et simultanément un éclaircissement de la fibre qui se traduit en pratique par la possibilité d'obtenir une coloration finale plus claire que la couleur d'origine. En outre, l'éclaircissement de la fibre a pour effet avantageux d'engendrer une couleur unie dans le cas des cheveux gris, et dans le cas de cheveux naturellement pigmentés, de faire ressortir la couleur, c'est-à-dire de la rendre plus visible.

Le procédé classiquement utilisé en coloration directe consiste à appliquer sur les fibres kératiniques des colorants directs, ou molécules colorantes, ayant une affinité pour lesdites fibres, à les laisser pauser, puis à rincer les fibres. Les colorants directs jusqu'ici utilisés sont des nitrés benzéniques, des colorants anthraquinoniques, des nitropyridines, des colorants de type azoïque, xanthénique, acridinique, azinique ou des colorants du triarylméthane. Les colorations qui en résultent sont des colorations temporaires ou semi-permanentes, car la nature des interactions qui lient les colorants directs à la fibre kératinique, et leur désorption de la surface et/ou du coeur de la fibre sont responsables de leur faible puissance tinctoriale et de leur mauvaise tenue aux lavages ou à la transpiration. Ces colorants directs sont en outre généralement sensibles à l'action des agents oxydants tels que l'eau oxygénée, ce qui les rend généralement inutilisables dans des compositions de teinture directe éclaircissantes à base d'eau oxygénée et d'un agent alcalinisant, lesquelles se rapprocheraient des teintures d'oxydation.
Ces colorants directs présentent également un certain manque de stabilité à la lumière lié à la faible résistance du chromophore vis-à-vis des attaques photochimiques. En outre, leur sensibilité à la lumière est dépendante de la répartition de leurs molécules, uniforme ou en aggrégats, dans le substrat.

Par conséquent, il existe un réel besoin de rechercher des colorants directs qui permettent de teindre les fibres kératiniques aussi puissamment que les colorants d'oxydation, qui soient aussi stables qu'eux à la lumière, soient également résistants aux intempéries, aux lavages et à la transpiration, et en outre, suffisamment stables en présence d'agents oxydants tels que l'eau oxygénée pour pouvoir obtenir un éclaircissement simultané de la fibre avec les avantages ci-avant exposés.

Dans la littérature, et notamment dans les revues Z. Chem.. (1985) 25(11), pages 402-403, et J. prakt. Chem. (1986) Vol 328 n°3 pages 342-348, au cours de la synthèse de 7-N,N-dialkylamino-1H-pyrazolo[3,4-b]-quinoxalines, L. HENNIG a synthétisé et caractérisé le composé 5-imino-4-[4'-(N,N-diéthylamino)-phényl]-imino-3-méthyl-1-phényl-pyrazoline et décrit la 5-imino-4-[4'-(N,N-diméthylamino)-phényl]-imino-3-méthyl-1-phénylpyrazoline. Par ailleurs, dans une thèse de l'Ecole Supérieure de Darmstadt de 1988, au cours de travaux relatifs à la synthèse organique de diaminopyrazoles , on a divulgué les composés 5-imino-4-[(2',4'-diamino)-phényl]-iminopyrazoline et 5-imino-4-[(2'-amino-4'-hydroxy-5'-méthyl)-phényl]-iminopyrazoline. Ces derniers composés sont colorés mais n'ont été décrits qu'à titre de colorants en tant que tels.
D'autre part, la demande de brevet WO-94/08971 décrit des dérivés pyrazoliques du triaminobenzène et les teintures les contenant.

Après d'importantes recherches menées sur la question, la demanderesse vient maintenant de découvrir une nouvelle famille de colorants directs, des 4,5-diiminopyrazolines ou leur forme tautomère 4-ylidèneamino-5-amino-pyrazoles, qui permettent de remédier aux inconvénients des colorants directs classiquement utilisés antérieurement, et conduisent à des teintures par coloration directe, dotées d'une très bonne résistance à la lumière, aux intempéries, aux lavages, à la transpiration et aux frottements. Leur bonne stabilité vis-à-vis des agents oxydants tels que l'eau oxygénée permet également de les utiliser dans un procédé de teinture directe éclaircissante.

Cette découverte est à la base de la présente invention.

La présente invention a ainsi pour objet une composition de teinture, comprenant, dans un milieu approprié pour la teinture, au moins un composé 4,5-diiminopyrazoline de formule (I) : 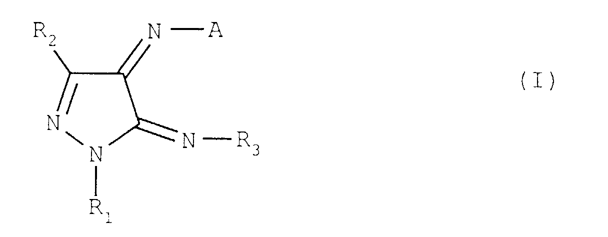 et/ou sa forme tautomère 4-ylidèneamino-5-amino-pyrazole de formule (II) : 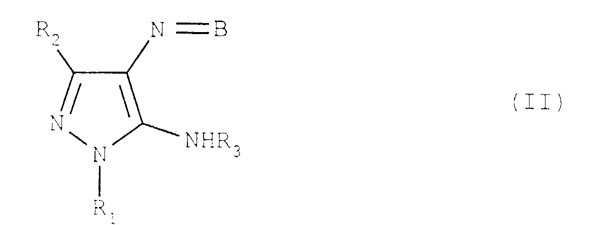 dans lesquelles,
- **R**_{**1**} et **R**_{**3**}, identiques ou différents, représentent un atome d'hydrogène, un radical alkyle en C₁-C₆, ou hydroxyalkyle en C₂-C₄, ou aminoalkyle en C₂-C₄, un radical phényle ou phényle substitué par un atome d'halogène ou par un radical alkyle en C₁-C₄, ou alcoxy en C₁-C₄, ou nitro, ou trifluorométhyle, ou amino, ou alkylamino en C₁-C₄, un radical benzyle ou benzyle substitué par un atome d'halogène ou par un radical alkyle en C₁-C₄, ou alcoxy en C₁-C₄, ou méthylènedioxy ou amino,
   un radical : 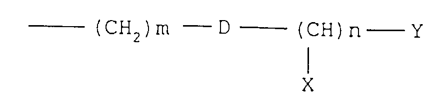 dans lequel m et n désignent un nombre entier allant de 1 à 3, inclusivement,
   D désigne oxygène ou -NH-,
   X désigne hydrogène ou CH₃,
   Y désigne CH₃ ou OR₄, R₄ désignant hydrogène, méthyle, ou éthyle,
   et R₃ peut également désigner un radical amino,
- **R**_{**2**} représente un atome d'hydrogène, un radical alkyle en C₁-C₆, ou hydroxyalkyle en C₁-C₄, ou aminoalkyle en C₁-C₄, ou alkylthio en C₁-C₂, ou alcoxy en C₁-C₅, ou trifluorométhyle, ou cyano, ou carboxyle, un radical phényle ou phényle substitué par un atome d'halogène ou par un radical alkyle en C₁-C₄, ou alcoxy en C₁-C₄, ou nitro, ou trifluorométhyle, ou amino, ou alkylamino en C₁-C₄, un radical benzyle ou benzyle substitué par un atome d'halogène ou par un radical alkyle en C₁-C₄, ou alcoxy en C₁-C₄, ou nitro, ou trifluorométhyle, ou amino, ou alkylamino en C₁-C₄, un hétérocycle choisi parmi le thiophène, le furane ou la pyridine,
   un radical :

   ―(CH₂)p―O―(CH₂)q―OR₅

   dans lequel p et q, identiques ou différents, sont des nombres entiers allant de 1 à 3 inclusivement, et R₅ désigne hydrogène ou méthyle,
- **A** représente, dans la formule (I), le radical : 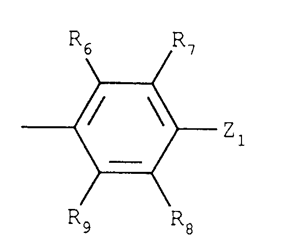 et,
- **B** représente dans la formule (II), le radical : 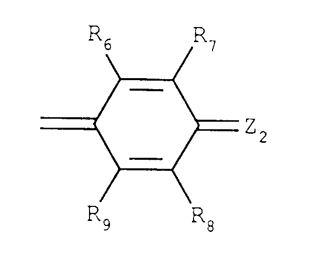 dans lesquels,
   **Z**_{**1**} représente un radical hydroxy ou alcoxy en C₁-C₄, ou amino, ou acétamido, ou alkylamino en C₁-C₄, ou hydroxyalkylamino en C₁-C₄, ou aminoalkylamino en C₁-C₄, un radical N R₁₀ R₁₁ dans lequel R₁₀ et R₁₁, identiques ou différents, désignent un radical alkyle en C₁-C₄, ou hydroxyalkyle en C₂-C₄ ou aminoalkyle en C₂-C₄.
   **R**_{**6**}**, R**_{**7**}**, R**_{**8**}**, R**_{**9**}, identiques ou différents, représentent, un atome d'hydrogène, un radical alkyle en C₁-C₄, ou alcoxy en C₁-C₄, ou alkylthio en C₁-C₄, un radical amino, alkylamino en C₁-C₄, ou carbamoylméthylamino, ou hydroxyalkyle en C₂-C₄, ou monohydroxyalcoxy en C₂-C₄, ou dihydroxyalcoxy en C₂-C₄, un radical carboxyalcoxy en C₁-C₂, un atome de chlore, ou de brome, ou de fluor, un radical trifluoroalkyle en C₁-C₂,
      un radical : 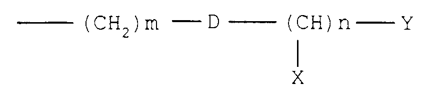
dans lequel, m, n, D, X, Y, ont les mêmes significations que ci-dessus, ou un radical N R₁₀ R₁₁ avec, pour R₁₀ et R₁₁ les mêmes significations que ci-dessus, **Z**_{**2**} représente O, ou NR avec R désignant H, ou acétyle, ou alkyle en C₁-C₄, ou hydroxyalkyle en C₁-C₄, ou aminoalkyle en C₁-C₄,
étant entendu que tous les radicaux alkyle ou alcoxy des groupements ci-avant définis peuvent être linéaires ou ramifiés, et sous réserve que,
(i) lorsque R₁ désigne phényle, que R₂ désigne méthyle, et que R₃ désigne hydrogène, A est différent de 4-diméthylaminophényle et de 4-diéthylaminophényle,
(ii) lorsque R₁, R₂, et R₃ désignent hydrogène, A est différent des radicaux 2-amino-4-hydroxy-5-méthylphényle et 2,4-diaminophényle.

Les composés de l'invention, lors de leur mise en oeuvre dans les formulations de teinture, répondent à l'une des structures de formule (I) et de formule (Il) ou peuvent, dans certains cas, se transformer d'une structure à l'autre selon un équilibre tautomérique. Cet équilibre tautomérique existe ou n'existe pas selon la nature des radicaux **Z**_{**1**} ou **Z**_{**2**} dans les formules (I) et (II).

Lesdits composés sont notamment choisis dans le groupe constitué par :
- le 5-imino-4-[4'-(N,N-diméthylamino)-phényl]-iminopyrazoline,
- le 5-imino-4-[4'-(N,N-diéthylamino)-phényl]-iminopyrazoline,
- le 5-imino-4-[4'-hydroxy-2'-amino-)-phényl]-iminopyrazoline,
- le 5-imino-4-[2',4'-diamino-5'-méthoxy)-phényl]-iminopyrazoline,
- le 5-imino-4-[(2'-(β-hydroxyéthyl)-amino-4'-hydroxy-5'-méthyl)-phényl]-iminopyrazoline,
- le 5-imino-4-[(2',4'-diamino-5'-(β-hydroxyéthyloxy))-phényl]-iminopyrazoline,
- le 5-imino-4-[4'-(N,N-diméthylamino)-phényl]-imino-1-méthyl-pyrazoline,
- le 5-imino-4-[4'-(N,N-diméthylamino)-phényl]-imino-1-éthyl-pyrazoline,
- le 5-imino-4-[4'-(N, N-diméthylamino)-phényl]-imino-1-isopropyl-pyrazoline,
- le 5-imino-4-[4'-(N,N-diméthylamino)-phényl]-imino-1-phényl-pyrazoline,
- le 5-imino-4-[4'-(N,N-diméthylamino)-phényl]-imino-1-terbutyl-pyrazoline,
- le 5-imino-4-[4'-(N,N-diméthylamino)-phényl]-imino-1-(β-hydroxyéthyl)-pyrazoline,
- le 5-imino-4-[4'-(N,N-diméthylamino)-phényl]-imino-1-benzyl-pyrazoline,
- le 5-imino-4-[4'-(N,N-diéthylamino)-phényl]-imino-1-méthyl-pyrazoline,
- le 5-imino-4-[4'-(N,N-diéthylamino)-phényl]-imino-1-éthyl-pyrazoline,
- le 5-imino-4-[4'-(N,N-diéthylamino)-phényl]-imino-1-isopropyl-pyrazoline,
- le 5-imino-4-[4'-(N,N-diéthylamino)-phényl]-imino-1-phényl-pyrazoline,
- le 5-imino-4-[4'-(N,N-diéthylamino)-phényl]-imino-1-terbutyl-pyrazoline,
- le 5-imino-4-[4'-(N,N-diéthylamino)-phényl]-imino-1-(β-hydroxyéthyl)-pyrazoline,
- le 5-imino-4-[4'-(N,N-diéthylamino)-phényl]-imino-1-benzyl-pyrazoline,
- le 5-imino-4-[2'-amino-4'-hydroxy)-phényl]-imino-1-méthyl-pyrazoline,
- le 5-imino-4-[2'-amino-4'-hydroxy)-phényl]-imino-1-éthyl-pyrazoline,
- le 5-imino-4-[2'-amino-4'-hydroxy)-phényl]-imino-1-isopropyl-pyrazoline,
- le 5-imino-4-[2'-amino-4'-hydroxy)-phényl]-imino-1-phényl-pyrazoline,
- le 5-imino-4-[2'-amino-4'-hydroxy)-phényl]-imino-1-benzyl-pyrazoline,
- le 5-imino-4-[2'-amino-4'-hydroxy)-phényl]-imino-1-terbutyl-pyrazoline,
- le 5-imino-4-[2'-amino-4'-hydroxy)-phényl]-imino-1-(β-hydroxyéthyl)-pyrazoline,
- le 5-imino-4-[2'-amino-4'-hydroxy-5'-méthyl)-phényl]-imino-1-méthyl-pyrazoline,
- le 5-imino-4-[2'-amino-4'-hydroxy-5'-méthyl)-phényl]-imino-1-éthyl-pyrazoline,
- le 5-imino-4-[2'-amino-4'-hydroxy-5'-méthyl)-phényl]-imino-1-isoproyl-pyrazoline,
- le 5-imino-4-[2'-amino-4'-hydroxy-5'-méthyl)-phényl]-imino-1-phényl-pyrazoline,
- le 5-imino-4-[2'-amino-4'-hydroxy-5'-méthyl)-phényl]-imino-1-benzyl-pyrazoline,
- le 5-imino-4-[2'-amino-4'-hydroxy-5'-méthyl)-phényl]-imino-1-terbutyl-pyrazoline,
- le 5-imino-4-[2'-amino-4'-hydroxy-5'-méthyl)-phényl]-imino-1-(β-hydroxyéthyl)-pyrazoline,
- le 5-imino-4-[2',4'-diamino-5'-méthoxy)-phényl]-imino-1-méthyl-pyrazoline,
- le 5-imino-4-[2',4'-diamino-5'-méthoxy)-phényl]-imino-1-éthyl-pyrazoline,
- le 5-imino-4-[2',4'-diamino-5'-méthoxy)-phényl]-imino-1-isopropyl-pyrazoline,
- le 5-imino-4-[2',4'-diamino-5'-méthoxy)-phényl]-imino-1-phényl-pyrazoline,
- le 5-imino-4-[2',4'-diamino-5'-méthoxy)-phényl]-imino-1benzyl-pyrazoline,
- le 5-imino-4-[2',4'-diamino-5'-méthoxy)-phényl]-imino-1-terbutyl-pyrazoline,
- le 5-imino-4-[2',4'-diamino-5'-méthoxy)-phényl]-imino-1-(β-hydroxyéthyl)-pyrazoline,
- le 5-imino-4-[(2'-(β-hydroxyéthyl)-amino-4'-hydroxy-5'-méthyl)-phényl]-imino-1-méthyl-pyrazoline,
- le 5-imino-4-[(2'-(β-hydroxyéthyl)-amino-4'-hydroxy-5'-méthyl)-phényl]-imino-1-éthyl-pyrazoline,
- le 5-imino-4-[(2'-(β-hydroxyéthyl)-amino-4'-hydroxy-5'-méthyl)-phényl]-imino-1-isopropyl-pyrazoline,
- le 5-imino-4-[(2'-(β-hydroxyéthyl)-amino-4'-hydroxy-5'-méthyl)-phényl]-imino-1-terbutyl-pyrazoline,
- le 5-imino-4-[(2'-(β-hydroxyéthyl)-amino-4'-hydroxy-5'-méthyl)-phényl]-imino-1-phényl-pyrazoline,
- le 5-imino-4-[(2'-(β-hydroxyéthyl)-amino-4'-hydroxy-5'-méthyl)-phényl]-imino-1-benzyl-pyrazoline,
- le 5-imino-4-[(2'-(β-hydroxyéthyl)-amino-4'-hydroxy-5'-méthyl)-phényl]-imino-1-(β-hydroxyéthyl)-pyrazoline,
- le 5-imino-4-[(2',4'-diamino-5'-(β-hydroxyéthyloxy))-phényl]-imino-1-méthyl-pyrazoline,
- le 5-imino-4-[(2',4'-diamino-5'-(β-hydroxyéthyloxy))-phényl]-imino-1-éthyl-pyrazoline,
- le 5-imino-4-[(2',4'-diamino-5'-(β-hydroxyéthyloxy))-phényl]-imino-1-isopropylpyrazoline,
- le 5-imino-4-[(2',4'-diamino-5'-(β-hydroxyéthyloxy))-phényl]-imino-1-terbutyl-pyrazoline,
- le 5-imino-4-[(2',4'-diamino-5'-(β-hydroxyéthyloxy))-phényl]-imino-1-phényl-pyrazoline,
- le 5-imino-4-[(2',4'-diamino-5'-(β-hydroxyéthyloxy))-phényl]-imino-1-benzyl-pyrazoline,
- le 5-imino-4-[(2',4'-diamino-5'-(β-hydroxyéthyloxy))-phényl]-imino-1-(β-hydroxyéthyl)-pyrazoline,
- le 5-imino-4-[4'-(N,N-diméthylamino)-phényl]-imino-3-méthyl-pyrazoline,
- le 5-imino-4-[4'-(N,N-diméthylamino)-phényl]-imino-3-méthyl-1-éthyl-pyrazoline,
- le 5-imino-4-[4'-(N,N-diméthylamino)-phényl]-imino-3-méthyl-1-isopropyl-pyrazoline,
- le 5-imino-4-[4'-(N,N-diméthylamino)-phényl]-imino-3-méthyl-1-terbutyl-pyrazoline,
- le 5-imino-4-[4'-(N,N-diméthylamino)-phényl]-imino-3-méthyl-1-phényl-pyrazoline,
- le 5-imino-4-[4'-(N,N-diméthylamino)-phényl]-imino-3-méthyl-1-benzyl-pyrazoline,
- le 5-imino-4-[4'-(N,N-diméthylamino)-phényl]-imino-3-méthyl-1-(β-hydroxyéthyl)-pyrazoline,
- le 5-imino-4-[4'-(N,N-diméthylamino)-phényl]-imino-1,3-diméthyl-pyrazoline,
- le 5-imino-4-[4'-(N,N-diéthylamino)-phényl]-imino-3-méthyl-pyrazoline,
- le 5-imino-4-[4'-(N, N-diéthylamino)-phényl]-imino-1,3-diméthyl-pyrazoline,
- le 5-imino-4-[4'-(N,N-diéthylamino)-phényl ]-imino-3-méthyl-1-éthyl-pyrazoline,
- le 5-imino-4-[4'-(N,N-diéthylamino)-phényl]-imino-3-méthyl-1-isopropyl-pyrazoline,
- le 5-imino-4-[4'-(N,N-diéthylamino)-phényl]-imino-3-méthyl-1-terbutyl-pyrazoline,
- le 5-imino-4-[4'-(N,N-diéthylamino)-phényl]-imino-3-méthyl-1-phényl-pyrazoline,
- le 5-imino-4-[4'-(N,N-diéthylamino)-phényl]-imino-3-méthyl-1-benzyl-pyrazoline,
- le 5-imino-4-[4'-(N,N-diéthylamino)-phényl]-imino-3-méthyl-1-(β-hydroxyéthyl)-pyrazoline,
- le 5-imino-4-[2'-amino-4'-hydroxy)-phényl]-imino-3-méthyl-pyrazoline,
- le 5-imino-4-[2'-amino-4'-hydroxy)-phényl]-imino-1,3-diméthyl-pyrazoline,
- le 5-imino-4-[2'-amino-4'-hydroxy)-phényl]-imino-3-méthyl-1-éthyl-pyrazoline,
- le 5-imino-4-[2'-amino-4'-hydroxy)-phényl]-imino-3-méthyl-1-isoproyl-pyrazoline,
- le 5-imino-4-[2'-amino-4'-hydroxy)-phényl]-imino-3-méthyl-1-terbutyl-pyrazoline,
- le 5-imino-4-[2'-amino-4'-hydroxy)-phényl]-imino-3-méthyl-1-phényl-pyrazoline,
- le 5-imino-4-[2'-amino-4'-hydroxy)-phényl]-imino-3-méthyl-1-benzyl-pyrazoline,
- le 5-imino-4-[2'-amino-4'-hydroxy)-phényl]-imino-3-méthyl-1-(β-hydroxyéthyl)-pyrazoline,
- le 5-imino-4-[2'-amino-4'-hydroxy-5'-méthyl)-phényl]-imino-3-méthyl-pyrazoline,
- le 5-imino-4-[2'-amino-4'-hydroxy-5'-méthyl)-phényl]-imino-1,3-diméthyl-pyrazoline,
- le 5-imino-4-[2'-amino-4'-hydroxy-5'-méthyl)-phényl]-imino-3-méthyl-1-éthyl-pyrazoline,
- le 5-imino-4-[2'-amino-4'-hydroxy-5'-méthyl)-phényl]-imino-3-méthyl-1-isoproylpyrazoline,
- le 5-imino-4-[2'-amino-4'-hydroxy-5'-méthyl)-phényl]-imino-3-méthyl-1-terbutylpyrazoline,
- le 5-imino-4-[2'-amino-4'-hydroxy-5'-méthyl)-phényl]-imino-3-méthyl-1-phénylpyrazoline,
- le 5-imino-4-[2'-amino-4'-hydroxy-5'-méthyl)-phényl]-imino-3-méthyl-1-benzylpyrazoline,
- le 5-imino-4-[2'-amino-4'-hydroxy-5'-methyl)-phényl]-imino-3-méthyl-1-(β-hydroxyéthyl)-pyrazoline,
- le 5-imino-4-[2',4'-diamino-5'-méthoxy)-phényl]-imino-3-méthyl-pyrazoline,
- le 5-imino-4-[2',4'-diamino-5'-méthoxy)-phényl]-imino-1,3-diméthyl-pyrazoline,
- le 5-imino-4-[2',4'-diamino-5'-méthoxy)-phényl]-imino-3-méthyl-1-éthyl-pyrazoline,
- le 5-imino-4-[2',4'-diamino-5'-méthoxy)-phényl]-imino-3-méthyl-1-isopropyl-pyrazoline,
- le 5-imino-4-[2',4'-diamino-5'-méthoxy)-phényl]-imino-3-méthyl-1-terbutyl-pyrazoline,
- le 5-imino-4-[2',4'-diamino-5'-méthoxy)-phényl]-imino-3-méthyl-1-phényl-pyrazoline,
- le 5-imino-4-[2',4'-diamino-5'-méthoxy)-phényl]-imino-3-méthyl-1-benzyl-pyrazoline,
- le 5-imino-4-[2',4'-diamino-5'-méthoxy)-phényl]-imino-3-méthyl-1-(β-hydroxyéthyl)-pyrazoline,
- le 5-imino-4-[2'-(β-hydroxyéthyl)-amino-4'-hydroxy-5'-méthyl)-phényl]-imino-3-méthyl-pyrazoline,
- le 5-imino-4-[2'-(β-hydroxyéthyl)-amino-4'-hydroxy-5'-méthyl)-phényl]-imino-1,3-diméthyl-pyrazoline,
- le 5-imino-4-[2'-(β-hydroxyéthyl)-amino-4'-hydroxy-5'-méthyl)-phényl]-imino-3-méthyl-1-éthyl-pyrazoline,
- le 5-imino-4-[2'-(β-hydroxyéthyl)-amino-4'-hydroxy-5'-méthyl)-phényl]-imino-3-méthyl-1-isopropyl-pyrazoline,
- le 5-imino-4-[2'-(β-hydroxyéthyl)-amino-4'-hydroxy-5'-méthyl)-phényl]-imino-3-méthyl-1-terbutyl-pyrazoline,
- le 5-imino-4-[2'-(β-hydroxyéthyl)-amino-4'-hydroxy-5'-méthyl)-phényl]-imino-3-méthyl-1-phényl-pyrazoline,
- le 5-imino-4-[2'-(β-hydroxyéthyl)-amino-4'-hydroxy-5'-méthyl)-phényl]-imino-3-méthyl-1-benzyl-pyrazoline,
- le 5-imino-4-[2'-(β-hydroxyéthyl)-amino-4'-hydroxy-5'-méthyl)-phényl]-imino-3-méthyl-1-(β-hydroxyéthyl)-pyrazoline,
- le 5-imino-4-[(2',4'-diamino-5'-(β-hydroxyéthyloxy))-phényl]-imino-3-méthyl-pyrazoline,
- le 5-imino-4-[2',4'-diamino-5'-(β-hydroxyéthyloxy))-phényl]-imino-1,3-diméthylpyrazoline,
- le 5-imino-4-[2',4'-diamino-5'-(β-hydroxyéthyloxy))-phényl]-imino-3-méthyl-1-éthyl-pyrazoline,
- le 5-imino-4-[2',4'-diamino-5'-(β-hydroxyéthyloxy))-phényl]-imino-3-méthyl-1-isopropyl-pyrazoline,
- le 5-imino-4-[2',4'-diamino-5'-(β-hydroxyéthyloxy))-phényl]-imino-3-méthyl-1-terbutyl-pyrazoline,
- le 5-imino-4-[2',4'-diamino-5'-(β-hydroxyéthyloxy))-phényl]-imino-3-méthyl-1-phényl-pyrazoline,
- le 5-imino-4-[2',4'-diamino-5'-(β-hydroxyéthyloxy))-phényl]-imino-3-méthyl-1-benzyl-pyrazoline,
- le 5-imino-4-[2',4'-diamino-5'-(β-hydroxyéthyloxy))-phényl]-imino-3-méthyl-1-(β-hydroxy-éthyl)-pyrazoline,
- le 3-hydroxyméthyl-5-imino4-[4'-(N,N-diméthylamino)-phényl]-iminopyrazoline,
- le 3-hydroxyméthyl-5-imino4-[4'-(N,N-diméthylamino)-phényl]-imino-1-méthyl-pyrazoline,
- le 3-hydroxyméthyl-5-imino-4-[4'-(N,N-diméthylamino)-phényl]-imino-1-éthylpyrazoline,
- le 3-hydroxyméthyl-5-imino-4-[4'-(N,N-diméthylamino)-phényl]-imino-1-isopropylpyrazoline,
- le 3-hydroxyméthyl-5-imino4-[4'-(N,N-diméthylamino)-phényl]-imino-1-terbutylpyrazoline,
- le 3-hydroxyméthyl-5-imino-4-[4'-(N,N-diméthylamino)-phényl]-imino-1-phénylpyrazoline,
- le 3-hydroxyméthyl-5-imino-4-[4'-(N,N-diméthylamino)-phényl]-imino-1-benzylpyrazoline,
- le 3-hydroxyméthyl-5-imino-4-[4'-(N,N-diméthylamino)-phényl]-imino-1-(β-hydroxyéthyl)-pyrazoline,
- le 3-hydroxyméthyl-5-imino-4-[4'-(N,N-diéthylamino)-phényl]-iminopyrazoline,
- le 3-hydroxyméthyl-5-imino-4-[4'-(N,N-diéthylamino)-phényl]-imino-1-méthylpyrazoline,
- le 3-hydroxyméthyl-5-imino-4-[4'-(N,N-diéthylamino)-phényl]-imino-1-éthylpyrazoline,
- le 3-hydroxyméthyl-5-imino-4-[4'-(N,N-diéthylamino)-phényl]-imino-1-isopropylpyrazoline,
- le 3-hydroxyméthyl-5-imino-4-[4'-(N,N-diéthylamino)-phényl]-imino-1-terbutylpyrazoline,
- le 3-hydroxyméthyl-5-imino-4-[4'-(N,N-diéthylamino)-phényl]-imino-1-phénylpyrazoline,
- le 3-hydroxyméthyl-5-imino-4-[4'-(N,N-diéthylamino)-phényl]-imino-1-benzylpyrazoline,
- le 3-hydroxyméthyl-5-imino4-[4'-(N,N-diéthylamino)-phényl]-imino-1-(β-hydroxyéthyl)-pyrazoline,
- le 3-hydroxyméthyl-5-imino-4-[(2'-amino-4'-hydroxy)-phényl]-iminopyrazoline,
- le 3-hydroxyméthyl-5-imino-4-[(2'-amino-4'-hydroxy)-phényl]-imino-1-méthylpyrazoline,
- le 3-hydroxyméthyl-5-imino-4-[(2'-amino-4'-hydroxy)-phényl]-imino-1-éthylpyrazoline,
- le 3-hydroxyméthyl-5-imino-4-[(2'-amino-4'-hydroxy)-phényl]-imino-1-isopropylpyrazoline,
- le 3-hydroxyméthyl-5-imino-4-[(2'-amino-4'-hydroxy)-phényl]-imino-1-terbutylpyrazoline,
- le 3-hydroxyméthyl-5-imino-4-[(2'-amino-4'-hydroxy)-phényl]-imino-1-phénylpyrazoline,
- le 3-hydroxyméthyl-5-imino-4-[(2'-amino-4'-hydroxy)-phényl]-imino-1-benzylpyrazoline,
- le 3-hydroxyméthyl-5-imino-4-[(2'-amino-4'-hydroxy)-phényl]-imino-1-(β-hydroxyéthyl)-pyrazoline,
- le 3-hydroxyméthyl-5-imino-4-[(2'-amino-4'-hydroxy-5'-méthyl)-phényl]-iminopyrazoline,
- le 3-hydroxyméthyl-5-imino-4-[(2'-amino-4'-hydroxy-5'-méthyl)-phényl]-imino-1-méthyl-pyrazoline,
- le 3-hydroxyméthyl-5-imino-4-[(2'-amino-4'-hydroxy-5'-méthyl)-phényl]-imino-1-éthyl-pyrazoline,
- le 1-hydroxyméthyl-5-imino-4-[(2'-amino-4'-hydroxy-5'-méthyl)-phényl]-imino-1-isopropyl-pyrazoline,
- le 3-hydroxyméthyl-5-imino-4-[(2'-amino-4'-hydroxy-5'-méthyl)-phényl]-imino-1-terbutyl-pyrazoline,
- le 3-hydroxyméthyl-5-imino-4-[(2'-amino-4'-hydroxy-5'-méthyl)-phényl]-imino-1-phényl-pyrazoline,
- le 3-hydroxyméthyl-5-imino-4-[(2'-amino-4'-hydroxy-5'-méthyl)-phényl]-imino-1-benzyl-pyrazoline,
- le 3-hydroxyméthyl-5-imino-4-[(2'-amino-4'-hydroxy-5'-méthyl)-phényl]-imino-1-(β-hydroxyéthyl)-pyrazoline,
- le 3-hydroxyméthyl-5-imino-4-[(2',4'-diamino-5'-méthoxy)-phényl]-imino-pyrazoline,
- le 3-hydroxyméthyl-5-imino-4-[(2',4'-diamino-5'-méthoxy)-phényl]-imino-1-méthyl-pyrazoline,
- le 3-hydroxyméthyl-5-imino-4-[(2',4'-diamino-5'-méthoxy)-phényl]-imino-1-éthylpyrazoline,
- le 3-hydroxyméthyl-5-imino-4-[(2',4'-diamino-5'-méthoxy)-phényl]-imino-1-isopropyl-pyrazoline,
- le 3-hydroxyméthyl-5-imino-4-[(2',4'-diamino-5'-méthoxy)-phényl]-imino-1-terbutyl-pyrazoline,
- le 3-hydroxyméthyl-5-imino-4-[(2',4'-diamino-5'-méthoxy)-phényl]-imino-1-phényl-pyrazoline,
- le 3-hydroxyméthyl-5-imino-4-[(2',4'-diamino-5'-méthoxy)-phényl]-imino-1-benzyl-pyrazoline,
- le 3-hydroxyméthyl-5-imino-4-[(2',4'-diamino-5'-méthoxy)-phényl]-imino-1-(β-hydroxyéthyl)-pyrazoline,
- le 3-hydroxyméthyl-5-imino-4-[(2',4'-diamino-5'-(β-hydroxyéthyloxy))-phényl]imino-pyrazoline,
- le 3-hydroxyméthyl-5-imino-4-[(2',4'-diamino-5'-(β-hydroxyéthyloxy))-phényl]imino-1-méthyl-pyrazoline,
- le 3-hydroxyméthyl-5-imino-4-[(2',4'-diamino-5'-(β-hydroxyéthyloxy))-phényl]-imino-1-éthyl-pyrazoline,
- le 3-hydroxyméthyl-5-imino-4-[(2',4'-diamino-5'-(β-hydroxyéthyloxy))-phényl]-imino-1-isopropyl-pyrazoline,
- le 3-hydroxyméthyl-5-imino-4-[(2',4'-diamino-5'-(β-hydroxyéthyloxy))-phényl]-imino-1-terbutyl-pyrazoline,
- le 3-hydroxyméthyl-5-imino-4-[(2',4'-diamino-5'-(β-hydroxyéthyloxy))-phényl]-imino-1-phényl-pyrazoline,
- le 3-hydroxyméthyl-5-imino-4-[(2',4'-diamino-5'-(β-hydroxyéthyloxy))-phényl]-imino-1-benzyl-pyrazoline,
- le 3-hydroxyméthyl-5-imino-4-[(2',4'-diamino-5'-(β-hydroxyéthyloxy))-phényl]-imino-1-(β-hydroxyéthyl)-pyrazoline,
- le 3-hydroxyméthyl-5-imino-4-[(2'-(β-hydroxyéthyl)-amino-4'-hydroxy-5'-méthyl)-phényl]-imino-pyrazoline,
- le 3-hydroxyméthyl-5-imino-4-[(2'-(β-hydroxyéthyl)-amino-4'-hydroxy-5'-méthyl)-phényl]-imino-1-méthyl-pyrazoline,
- le 3-hydroxyméthyl-5-imino-4-[(2'-(β-hydroxyéthyl)-amino-4'-hydroxy-5'-méthyl)-phényl]-imino-1-éthyl-pyrazoline,
- le 3-hydroxyméthyl-5-imino-4-[(2'-(β-hydroxyéthyl)-amino-4'-hydroxy-5'-méthyl)-phényl]-imino-1-isopropyl-pyrazoline,
- le 3-hydroxyméthyl-5-imino-4-[(2'-(β-hydroxyéthyl)-amino-4'-hydroxy-5'-méthyl)-phényl]-imino-1-terbutyl-pyrazoline,
- le 3-hydroxyméthyl-5-imino-4-[(2'-(β-hydroxyéthyl)-amino-4'-hydroxy-5'-méthyl)-phényl]-imino-1-phényl-pyrazoline,
- le 3-hydroxyméthyl-5-imino-4-[(2'-(β-hydroxyéthyl)-amino-4'-hydroxy-5'-méthyl)-phényl]-imino-1-benzyl-pyrazoline,
- le 3-hydroxyméthyl-5-imino-4-[(2'-(β-hydroxyéthyl)-amino-4'-hydroxy-5'-méthyl)-phényl]-imino-1-(β-hydroxyéthyl)-pyrazoline,
- le 3-trifluorométhyl-5-imino-4-[4'-(N,N-diméthylamino)-phényl]-iminopyrazoline,
- le 3-trifluorométhyl-5-imino-4-[4'-(N,N-diméthylamino)-phényl]-imino-1-méthyl-pyrazoline,
- le 3-trifluorométhyl-5-imino-4-[4'-(N,N-diméthylamino)-phényl]-imino-1-éthyl-pyrazoline,
- le 3-trifluorométhyl-5-imino-4-[4'-(N,N-diméthylamino)-phényl]-imino-1-isopropyl-pyrazoline,
- le 3-trifluorométhyl-5-imino-4-[4'-(N,N-diméthylamino)-phényl]-imino-1-terbutyl-pyrazoline,
- le 3-trifluorométhyl-5-imino-4-[4'-(N,N-diméthylamino)-phényl]-imino-1-phényl-pyrazoline,
- le 3-trifluorométhyl-5-imino-4-[4'-(N,N-diméthylamino)-phényl]-imino-1-benzyl-pyrazoline,
- le 3-trifluorométhyl-5-imino-4-[4'-(N,N-diméthylamino)-phényl]-imino-1-(β-hydroxyéthyl)-pyrazoline,
- le 3-trifluorométhyl-5-imino-4-[4'-(N,N-diéthylamino)-phényl]-iminopyrazoline,
- le 3-trifluorométhyl-5-imino-4-[4'-(N,N-diéthylamino)-phényl]-imino-1-méthyl-pyrazoline,
- le 3-trifluorométhyl-5-imino-4-[4'-(N,N-diéthylamino)-phényl]-imino-1-éthyl-pyrazoline,
- le 3-trifluorométhyl-5-imino-4-[4'-(N,N-diéthylamino)-phényl]-imino-1-isopropyl-pyrazoline,
- le 3-trifluorométhyl-5-imino-4-[4'-(N,N-diéthylamino)-phényl]-imino-1-terbutyl-pyrazoline,
- le 3-trifluorométhyl-5-imino-4-[4'-(N,N-diéthylamino)-phényl]-imino-1-phényl-pyrazoline,
- le 3-trifluorométhyl-5-imino-4-[4'-(N,N-diéthylamino)-phényl]-imino-1-benzyl-pyrazoline,
- le 3-trifluorométhyl-5-imino-4-[4'-(N,N-diéthylamino)-phényl]-imino-1-(β-hydroxyéthyl)-pyrazoline,
- le 3-trifluorométhyl-5-imino4-[(2'-amino-4'-hydroxy)-phényl]-iminopyrazoline,
- le 3-trifluorométhyl-5-imino-4-[(2'-amino-4'-hydroxy)-phényl]-imino-1-méthyl-pyrazoline,
- le 3-trifluorométhyl-5-imino-4-[(2'-amino-4'-hydroxy)-phényl]-imino-1-éthyl-pyrazoline,
- le 3-trifluorométhyl-5-imino-4-[(2'-amino-4'-hydroxy)-phényl]-imino-1-isopropyl-pyrazoline,
- le 3-trifluorométhyl-5-imino-4-[(2'-amino-4'-hydroxy)-phényl]-imino-1-terbutyl-pyrazoline,
- le 3-trifluorométhyl-5-imino-4-[(2'-amino-4'-hydroxy)-phényl]-imino-1-phényl-pyrazoline,
- le 3-trifluorométhyl-5-imino-4-[(2'-amino-4'-hydroxy)-phényl]-imino-1-benzyl-pyrazoline,
- le 3-trifluorométhyl-5-imino-4-[(2'-amino-4'-hydroxy)-phényl]-imino-1-(β-hydroxyéthyl)-pyrazoline,
- le 3-trifluorométhyl-5-imino-4-[(2'-amino-4'-hydroxy-5'-méthyl)-phényl]-imino-pyrazoline,
- le 3-trifluorométhyl-5-imino-4-[(2'-amino-4'-hydroxy-5'-méthyl)-phényl]-imino-1-méthyl-pyrazoline,
- le 3-trifluorométhyl-5-imino-4-[(2'-amino-4'-hydroxy-5'-méthyl)-phényl]-imino-1-éthyl-pyrazoline,
- le 3-trifluorométhyl-5-imino-4-[(2'-amino-4'-hydroxy-5'-méthyl)-phényl]-imino-1-isopropyl-pyrazoline,
- le 3-trifluorométhyl-5-imino-4-[(2'-amino-4'-hydroxy-5'-méthyl)-phényl]-imino-1-terbutyl-pyrazoline,
- le 3-trifluorométhyl-5-imino-4-[(2'-amino-4'-hydroxy-5'-méthyl)-phényl]-imino-1-phényl-pyrazoline,
- le 3-trifluorométhyl-5-imino-4-[(2'-amino-4'-hydroxy-5'-méthyl)-phényl]-imino-1-benzyl-pyrazoline,
- le 3-trifluorométhyl-5-imino-4-[(2'-amino-4'-hydroxy-5'-méthyl)-phényl]-imino-1-(β-hydroxyéthyl)-pyrazoline,
- le 3-trifluorométhyl-5-imino-4-[(2',4'-diamino-5'-méthoxy)-phényl]-imino-pyrazoline,
- le 3-trifluorométhyl-5-imino-4-[(2',4'-diamino-5'-méthoxy)-phényl]-imino-1-méthyl-pyrazoline,
- le 3-trifluorométhyl-5-imino-4-[(2',4'-diamino-5'-méthoxy)-phényl]-imino-1-éthyl-pyrazoline,
- le 3-trifluorométhyl-5-imino-4-[(2',4'-diamino-5'-méthoxy)-phényl]-imino-1-isopropyl-pyrazoline,
- le 3-trifluorométhyl-5-imino-4-[(2',4'-diamino-5'-méthoxy)-phényl]-imino-1-terbutyl-pyrazoline,
- le 3-trifluorométhyl-5-imino-4-[(2',4'-diamino-5'-méthoxy)-phényl]-imino-1-phényl-pyrazoline,
- le 3-trifluorométhyl-5-imino-4-[(2',4'-diamino-5'-méthoxy)-phényl]-imino-1-benzyl-pyrazoline,
- le 3-trifluorométhyl-5-imino-4-[(2',4'-diamino-5'-méthoxy)-phényl]-imino-1-(β-hydroxyéthyl)-pyrazoline,
- le 3-trifluorométhyl-5-imino-4-[(2',4'-diamino-5'-(β-hydroxyéthyloxy))-phényl]-imino-pyrazoline,
- le 3-trifluorométhyl-5-imino-4-[(2',4'-diamino-5'-(β-hydroxyéthyloxy))-phényl]-imino-1-méthyl-pyrazoline,
- le 3-trifluorométhyl-5-imino-4-[(2',4'-diamino-5'-(β-hydroxyéthyloxy))-phényl]-imino-1-éthyl-pyrazoline,
- le 3-trifluorométhyl-5-imino-4-[(2',4'-diamino-5'-(β-hydroxyéthyloxy))-phényl]-imino-1-isopropyl-pyrazoline,
- le 3-trifluorométhyl-5-imino4-[(2',4'-diamino-5'-(β-hydroxyéthyloxy))-phényl]-imino-1-terbutyl-pyrazoline,
- le 3-trifluorométhyl-5-imino4-[(2',4'-diamino-5'-(β-hydroxyéthyloxy))-phényl]-imino-1-phényl-pyrazoline,
- le 3-trifluorométhyl-5-imino-4-[(2',4'-diamino-5'-(β-hydroxyéthyloxy))-phényl]-imino-1-benzyl-pyrazoline,
- le 3-trifluorométhyl-5-imino-4-[(2',4'-diamino-5'-(β-hydroxyéthyloxy))-phényl]-imino-1-(β-hydroxyéthyl)-pyrazoline,
- le 3-trifluorométhyl-5-imino-4-[(2'-(β-hydroxyéthyl)-amino-4'-hydroxy-5'-méthyl)-phényl]-imino-pyrazoline,
- le 3-trifluorométhyl-5-imino-4-[(2'-(β-hydroxyéthyl)-amino-4'-hydroxy-5'-méthyl)-phényl]-imino-1-méthyl-pyrazoline,
- le 3-trifluorométhyl-5-imino-4-[(2'-(β-hydroxyéthyl)-amino-4'-hydroxy-5'-méthyl)-phényl]-imino-1-éthyl-pyrazoline,
- le 3-trifluorométhyl-5-imino-4-[(2'-(β-hydroxyéthyl)-amino-4'-hydroxy-5'-méthyl)-phényl]-imino-1-isopropyl-pyrazoline,
- le 3-trifluorométhyl-5-imino-4-[(2'-(β-hydroxyéthyl)-amino-4'-hydroxy-5'-méthyl)-phényl]-imino-1-terbutyl-pyrazoline,
- le 3-trifluorométhyl-5-imino-4-[(2'-(β-hydroxyéthyl)-amino-4'-hydroxy-5'-méthyl)-phényl]-imino-1-phényl-pyrazoline,
- le 3-trifluorométhyl-5-imino-4-[(2'-(β-hydroxyéthyl)-amino-4'-hydroxy-5'-méthyl)-phényl]-imino-1-benzyl-pyrazoline,
- le 3-trifluorométhyl-5-imino-4-[(2'-(β-hydroxyéthyl)-amino-4'-hydroxy-5'-méthyl)-phényl]-imino-1-(β-hydroxyéthyl)-pyrazoline,
- le 3-diméthylamino-5-imino-4-[4'-(N,N-diméthylamino)-phényl]-iminopyrazoline,
- le 3-diméthylamino-5-imino-4-[4'-(N,N-diméthylamino)-phényl]-imino-1-méthyl-pyrazoline,
- le 3-diméthylamino-5-imino-4-[4'-(N,N-diméthylamino)-phényl]-imino-1-éthyl-pyrazoline,
- le 3-diméthylamino-5-imino-4-[4'-(N,N-diméthylamino)-phényl]-imino-1-isopropyl-pyrazoline,
- le 3-diméthylamino-5-imino-4-[4'-(N,N-diméthylamino)-phényl]-imino-1-terbutyl-pyrazoline,
- le 3-diméthylamino-5-imino-4-[4'-(N,N-diméthylamino)-phényl]-imino-1-phényl-pyrazoline,
- le 3-diméthylamino-5-imino-4-[4'-(N,N-diméthylamino)-phényl]-imino-1-benzyl-pyrazoline,
- le 3-diméthylamino-5-imino-4-[4'-(N,N-diméthylamino)-phényl]-imino-1-β-hydroxyéthyl)-pyrazoline,
- le 3-diméthylamino-5-imino-4-[4'-(N,N-diéthylamino)-phényl]-iminopyrazoline,
- le 3-diméthylamino-5-imino-4-[4'-(N,N-diéthylamino)-phényl]-imino-1-méthyl-pyrazoline,
- le 3-diméthylamino-5-imino-4-[4'-(N,N-diéthylamino)-phényl]-imino-1-éthyl-pyrazoline,
- le 3-diméthylamino-5-imino-4-[4'-(N,N-diéthylamino)-phényl]-imino-1-isopropyl-pyrazoline,
- le 3-diméthylamino-5-imino-4-[4'-(N,N-diéthylamino)-phényl]-imino-1-terbutyl-pyrazoline,
- le 3-diméthylamino-5-imino-4-[4'-(N,N-diéthylamino)-phényl]-imino-1-phényl-pyrazoline,
- le 3-diméthylamino-5-imino-4-[4'-(N,N-diéthylamino)-phényl]-imino-1-benzyl-pyrazoline,
- le 3-diméthylamino-5-imino-4-[4'-(N,N-diéthylamino)-phényl]-imino-1-(β-hydroxyéthyl)-pyrazoline,
- le 3-diméthylaminométhyl-5-imino-4-[(4'-hydroxy-2'-amino)-phényl]-imino-pyrazoline,
- le 3-diméthylaminométhyl-5-imino-4-[(4'-hydroxy-2'-amino)-phényl]-imino-1-méthyl-pyrazoline,
- le 3-diméthylaminométhyl-5-imino-4-[(4'-hydroxy-2'-amino)-phényl]-imino-1-éthyl-pyrazoline,
- le 3-diméthylaminométhyl-5-imino-4-[(4'-hydroxy-2'-amino)-phényl]-imino-1-isopropyl-pyrazoline,
- le 3-diméthylaminométhyl-5-imino-4-[(4'-hydroxy-2'-amino)-phényl]-imino-1-terbutyl-pyrazoline,
- le 3-diméthylaminométhyl-5-imino-4-[(4'-hydroxy-2'-amino)-phényl]-imino-1-phényl-pyrazoline,
- le 3-diméthylaminométhyl-5-imino-4-[(4'-hydroxy-2'-amino)-phényl]-imino-1-benzyl-pyrazoline,
- le 3-diméthylaminométhyl-5-imino-4-[(4'-hydroxy-2'-amino)-phényl]-imino-1-(β-hydroxyéthyl)-pyrazoline,
- le 3-diméthylaminométhyl-5-imino-4-[(2'-amino-4'-hydroxy-5'-méthyl)-phényl]-imino-pyrazoline,
- le 3-diméthylaminométhyl-5-imino-4-[(2'-amino-4'-hydroxy-5'-méthyl)-phényl]-imino-1-méthyl-pyrazoline,
- le 3-diméthylaminométhyl-5-imino-4-[(2'-amino-4'-hydroxy-5'-méthyl)-phényl]-imino-1-éthyl-pyrazoline,
- le 3-diméthylaminométhyl-5-imino-4-[(2'-amino-4'-hydroxy-5'-méthyl)-phényl]-imino-1-isopropyl-pyrazoline,
- le 3-diméthylaminométhyl-5-imino-4-[(2'-amino-4'-hydroxy-5'-méthyl)-phényl]-imino-1-terbutyl-pyrazoline,
- le 3-diméthylaminométhyl-5-imino-4-[(2'-amino-4'-hydroxy-5'-méthyl)-phényl]-imino-1-phényl-pyrazoline,
- le 3-diméthylaminométhyl-5-imino-4-[(2'-amino-4'-hydroxy-5'-méthyl)-phényl]-imino-1-benzyl-pyrazoline,
- le 3-diméthylaminométhyl-5-imino-4-[(2'-amino-4'-hydroxy-5'-méthyl)-phényl]-imino-1-(β-hydroxyéthyl)-pyrazoline,
- le 3-diméthylaminométhyl-5-imino-4-[(2',4'-diamino-5'-méthoxy)-phényl]-imino-pyrazoline,
- le 3-diméthylaminométhyl-5-imino-4-[(2',4'-diamino-5'-méthoxy)-phényl]-imino-1-méthyl-pyrazoline,
- le 3-diméthylaminométhyl-5-imino-4-[(2',4'-diamino-5'-methoxy)-phényl]-imino-1-éthyl-pyrazoline,
- le 3-diméthylaminométhyl-5-imino-4-[(2',4'-diamino-5'-méthoxy)-phényl]-imino-1-isopropyl-pyrazoline,
- le 3-diméthylaminométhyl-5-imino-4-[(2',4'-diamino-5'-méthoxy)-phényl]-imino-1-terbutyl-pyrazoline,
- le 3-diméthylaminométhyl-5-imino-4-[(2',4'-diamino-5'-méthoxy)-phényl]-imino-1-phényl-pyrazoline,
- le 3-diméthylaminométhyl-5-imino-4-[(2',4'-diamino-5'-méthoxy)-phényl]-imino-1-benzyl-pyrazoline,
- le 3-diméthylaminométhyl-5-imino-4-[(2',4'-diamino-5'-méthoxy)-phényl]-imino-1-(β-hydroxyéthyl)-pyrazoline,
- le 3-diméthylaminométhyl-5-imino-4-[(2'-(β-hydroxyéthyl)-amino-4'-hydroxy-5'-méthyl)-phényl]-imino-pyrazoline,
- le 3-diméthylaminométhyl-5-imino-4-[(2'-(β-hydroxyéthyl)-amino-4'-hydroxy-5'-méthyl)-phényl]-imino-1-méthyl-pyrazoline,
- le 3-diméthylaminométhyl-5-imino-4-[(2'-(β-hydroxyéthyl)-amino-4'-hydroxy-5'-méthyl)-phényl]-imino-1-éthyl-pyrazoline,
- le 3-diméthylaminométhyl-5-imino-4-[(2'-(β-hydroxyéthyl)-amino-4'-hydroxy-5'-méthyl)-phényl]-imino-1-isopropyl-pyrazoline,
- le 3-diméthylaminométhyl-5-imino-4-[(2'-(β-hydroxyéthyl)-amino-4'-hydroxy-5'-méthyl)-phényl]-imino-1-terbutyl-pyrazoline,
- le 3-diméthylaminométhyl-5-imino-4-[(2'-(β-hydroxyéthyl)-amino-4'-hydroxy-5'-méthyl)-phényl]-imino-1-phényl-pyrazoline,
- le 3-diméthylaminométhyl-5-imino-4-[(2'-(β-hydroxyéthyl)-amino-4'-hydroxy-5'-méthyl)-phényl]-imino-1-benzyl-pyrazoline,
- le 3-diméthylaminométhyl-5-imino-4-[(2'-(β-hydroxyéthyl)-amino-4'-hydroxy-5'-méthyl)-phényl]-imino-1-(β-hydroxyéthyl)-pyrazoline,
- le 3-diméthylaminométhyl-5-imino-4-[(2',4'-diamino-5'-(β-hydroxyéthyloxy))-phényl]-imino-pyrazoline,
- le 3-diméthylaminométhyl-5-imino-4-[(2',4'-diamino-5'-(β-hydroxyéthyloxy))-phényl]-imino-1-méthyl-pyrazoline,
- le 3-diméthylaminométhyl-5-imino-4-[(2',4'-diamino-5'-(β-hydroxyéthyloxy))-phényl]-imino-1-éthyl-pyrazoline,
- le 3-diméthylaminométhyl-5-imino-4-[(2',4'-diamino-5'-(β-hydroxyéthyloxy))-phényl]-imino-1-isopropyl-pyrazoline,
- le 3-diméthylaminométhyl-5-imino-4-[(2',4'-diamino-5'-(β-hydroxyéthyloxy))-phényl]-imino-1-terbutyl-pyrazoline,
- le 3-diméthylaminométhyl-5-imino-4-[(2',4'-diamino-5'-(β-hydroxyéthyloxy))-phényl]-imino-1-phényl-pyrazoline,
- le 3-diméthylaminométhyl-5-imino-4-[(2',4'-diamino-5'-(β-hydroxyéthyloxy))-phényl]-imino-1-benzyl-pyrazoline,
- le 3-diméthylaminométhyl-5-imino-4-[(2',4'-diamino-5'-(β-hydroxyéthyloxy))-phényl]-imino-1-(β-hydroxyéthyl)-pyrazoline,
ainsi que leur forme tautomère 4-ylidèneamino-5-amino-pyrazole correspondante ou leurs mélanges.

Parmi les composés de l'invention plus particulièrement préférés, on peut citer :
- le 5-imino-4-[(2'-amino-4'-hydroxy-5'-méthyl)-phényl]-imino-1,3-diméthyl-pyrazoline,
- le 5-imino-4-[(2'-amino-4'-hydroxy-5'-méthyl)-phényl]-imino-pyrazoline,
- le 5-imino-4-[(2'-amino-4'-hydroxy-5'-méthyl)-phényl]-imino-1-méthyl-pyrazoline,
- le 5-imino-4-[(2'-amino-4'-hydroxy-5'-méthyl)-phényl]-imino-3-méthyl-pyrazoline,
- le 5-imino-4-[(2'-amino-4'-hydroxy)-phényl]-imino-1,3-diméthyl-pyrazoline,
- le 5-imino-4-[(2'-amino-4'-hydroxy)-phényl]-imino-1-méthyl-pyrazoline,
- le 5-imino-4-[(2'-amino-4'-hydroxy)-phényl]-imino-pyrazoline,
- le 5-imino-4-[(2'-amino-4'-hydroxy)-phényl]-imino-3-méthyl-pyrazoline,
- le 1-éthyl-5-imino-4-[(2'-amino-4'-hydroxy-5'-méthyl)-phényl]-imino-3-méthyl-pyrazoline,
- le 1-éthyl-5-imino-4-[(2'-amino-4'-hydroxy-5'-méthyl)-phényl]-imino-pyrazoline,
- le 1-éthyl-5-imino-4-[(2'-amino-4'-hydroxy)-phényl]-imino-3-méthyl-pyrazoline,
- le 1-éthyl-5-imino-4-[(2'-amino-4'-hydroxy)-phényl]-imino-pyrazoline,
- le 1-isoprepyl-5-imine-4-[(2'-amine-4'-hydroxy-5'-méthyl)-phényl]-imino-pyrazoline,
- le 1-isopropyl-5-imino-4-[(2'-amino-4'-hydroxy-5'-méthyl)-phényl]-imino-3-méthyl-pyrazoline,
- le 1-isopropyl-5-imino-4-[(2'-amino-4'-hydroxy)-phényl]-imino-pyrazoline,
- le 1-isopropyl-5-imino-4-[(2'-amino-4'-hydroxy)-phényl]-imino-3-méthyl-pyrazoline,
- le 1-(β-hydroxyéthyl)-5-imino-4-[(2'-amino-4'-hydroxy)-phényl]-imino-pyrazoline,
- le 1-(β-hydroxyéthyl)-5-imino-4-[(2'-amino-4'-hydroxy)-phényl]-imino-3-méthyl-pyrazoline,
- le 1-(β-hydroxyéthyl)-5-imino-4-[(2'-amino-4'-hydroxy-5'-méthyl)-phényl]-imino-pyrazoline,
- le 1-(β-hydroxyéthyl)-5-imino-4-[(2'-amino-4'-hydroxy-5'-méthyl)-phényl]-imino-3-méthyl-pyrazoline,
- le 5-imino-4-[(2'-amino-4'-hydroxy-5'-méthyl)-phényl]-imino-3-trifluorométhyl-pyrazoline,
- le 1-méthyl-5-imino-4-[(2'-amino-4'-hydroxy-5'-méthyl)-phényl]-imino-3-trifluorométhyl-pyrazoline,
- le 1-méthyl-5-imino-4-[(2'-amino-4'-hydroxy)-phényl]-imino-3-trifluorométhyl-pyrazoline,
- le 5-imino-4-[(2'-amino-4'-hydroxy)-phényl]-imino-3-trifluorométhyl-pyrazoline,
- le 1-éthyl-5-imino-4-[(2'-amino-4'-hydroxy-5'-méthyl)-phényl]-imino-3-trifluoro-méthyl-pyrazoline,
- le 1-éthyl-5-imino-4-[(2'-amino-4'-hydroxy)-phényl]-imino-3-trifluorométhyl-pyrazoline,
- le 1-isopropyl-5-imino-4-[(2'-amino-4'-hydroxy-5'-méthyl)-phényl]-imino-3-trifluorométhyl-pyrazoline,
- le 1-isopropyl-5-imino-4-[(2'-amino-4'-hydroxy)-phényl]-imino-3-trifluorométhyl-pyrazoline,
- le 1-(β-hydroxyéthyl)-5-imino-4-[(2'-amino-4'-hydroxy)-phényl]-imino-3-trifluorométhyl-pyrazoline,
- le 1-(β-hydroxyéthyl)-5-imino-4-[(2'-amino-4'-hydroxy-5'-méthyl)-phényl]-imino-3-trifluorométhyl-pyrazoline,
ainsi que leur forme tautomère 4-ylidèneamino-5-amino-pyrazole correspondante, ou leurs mélanges, lorsque l'équilibre tautomérique est possible.

L'invention a pour autre objet l'utilisation des composés de formule (I) ou (II) à titre de colorant direct, dans des, ou pour la préparation de, compositions tinctoriales pour fibres kératiniques.

L'invention a également pour objet une composition de teinture directe pour fibres kératiniques, en particulier pour fibres kératiniques humaines, telles que les cheveux, comprenant dans un milieu approprié pour la teinture, au moins un colorant direct tel que défini ci-avant par la formule (I) ou (II).

L'invention a aussi pour objet une composition de teinture éclaircissante pour fibres kératiniques, en particulier pour fibres kératiniques humaines, telles que les cheveux, prête à l'emploi, caractérisée par le fait qu'elle contient, dans un milieu approprié pour la teinture, au moins un colorant direct de formule (I) ou (II) ci-avant définie, avec un agent oxydant, et en outre un agent alcalinisant en quantité suffisante pour ajuster le pH final à une valeur supérieure à 7, et de préférence comprise entre les valeurs 8,5 et 11.

Mais d'autres caractéristiques, aspects et avantages de l'invention apparaîtront encore plus clairement à la lecture de la description qui va suivre, ainsi que des divers exemples concrets, mais nullement limitatifs, destinés à l'illlustrer.

La concentration en composé de formule (I) ou (II) est de préférence comprise entre 0,001 et 5%, et encore plus préférentiellement entre 0,01 et 3% en poids, par rapport au poids total de la composition tinctoriale, avant application sur les fibres.

La composition tinctoriale selon l'invention peut encore contenir, pour obtenir des teintes variées, outre les colorants de formule (I) ou (II), d'autre(s) colorant(s) direct(s) classiquement utilisés, et notamment, des colorants nitrés benzéniques, comme les nitrophénylènediamines, les nitrodiphénylamines, les nitroanilines, les éthers de phénol nitrés ou les nitrophénols, des nitropyridines, des colorants anthraquinoniques, mono- ou di-azoïques, triarylméthaniques, aziniques, acridiniques et xanthéniques, ou encore des colorants métallifères.

Parmi les colorants nitrés benzéniques utilisables en association avec les colorants de formule (I) ou (II), on préfère utiliser:
- le 3-amino-4-hydroxy-nitrobenzène,
- le 2-amino-3-hydroxy-nitrobenzène,
- le 2-amino-4-hydroxy-nitrobenzène,
- le 2-amino-5-hydroxy-nitrobenzène,
- le 5-amino-2-hydroxy-nitrobenzène,
- le 2-amino-5-N,N-bis-(β-hydroxyéthyl)-amino-nitrobenzène,
- le 2-amino-4-chloro-5-N-(β-hydroxyéthyl)-amino-nitrobenzène,
- le 2-amino-4-méthyl-5-N-(β-hydroxyéthyl)-amino-nitrobenzène,
- le 3,4-bis-N-(β-hydroxyéthyl)-amino-nitrobenzène,
- le 2-amino-4-méthyl-5-N-(β-γ-dihydroxypropyl)-amino-nitrobenzène,
- le 2-amino-4-méthyl-5-N-(β-aminoéthyl)-amino-nitrobenzène,
- le 1-amino-2-[tris(hydroxyméthyl)-méthyl]-amino-5-nitro-benzène,
- le 1-hydroxy-2-N-(β-hydroxyéthyl)-amino-4,6-dinitro-benzène,
- le 4-N-(β-hydroxyéthyl)-amino-3-nitro-chlorobenzène,
- le 4-N,N-bis-(β-hydroxyéthyl)-amino-1-N-(β-méthoxyéthyl)-amino-2-nitro-benzène,
- le 1-N-(β-γ-dihydroxypropyl)-amino-4-N-éthyl,N-(β-hydroxyéthyl)-amino-2-nitro-benzène,
- le 1-N-(β-γ-dihydroxypropyl)-amino-4-N-méthyl,N-(β-hydroxyéthyl)-amino-2-nitro-benzène,
- le 1-N-méthylamino-4-N-méthyl,N-(β-γ-dihydroxypropyl)-amino-2-nitro-benzène,
- le 2-chloro-6-éthylamino-1-hydroxy-nitrobenzène,
- le 2-N,N-bis-(β-hydroxyéthyl)-amino-1-hydroxy-5-nitro-benzène,
- le 1-N-(β-hydroxyéthyl)-amino-2-nitro-4-trifluorométhyl-benzène,
- l'acide 4-éthylamino-3-nitro-benzoïque,
- l'acide 4-amino-2-nitro-diphénylamine-2'-carboxylique,
- l'acide 4-amino-4'-diméthylamino-2-nitro-diphénylamine-2'-carboxylique,
et plus particulièrement encore,
- le 3,4-diamino-nitrobenzène,
- le 2,5-diamino-nitrobenzène,
- le 2-amino-1-hydroxy-4,6-dinitro-benzène,
- le 2-amino-5-N-(β-hydroxyéthyl)-amino-nitrobenzène,
- le 2-N-(β-hydroxyéthyl)-amino-5-N, N-bis-(β-hydroxyéthyl)-amino-nitrobenzène,
- le 2-N-méthylamino-5-N, N-bis-(β-hydroxyéthyl)-amino-nitrobenzène,
- le 2-N-méthylamino-5-N-méthyl,N-(β-hydroxyéthyl)-amino-nitrobenzène,
- le 2-N-(β-hydroxyéthyl)-amino-5-hydroxy-nitrobenzène,
- le 4-N-(β-hydroxyéthyl)-amino-3-méthoxy-nitrobenzène,
- le 2-N-(β-hydroxyéthyl)-amino-5-amino-nitrobenzène,
- le 2-N-(β-hydroxyéthyl)-amino-nitrobenzène,
- le 3-amino-4-N-(β-hydroxyéthyl)-amino-nitrobenzène,
- le 3-β-hydroxyéthyloxy-4-N-(β-hydroxyéthyl)-amino-nitrobenzène,
- le 2-amino-5-N-méthylamino-nitrobenzène,
- le 2-amino-3-méthyl-nitrobenzène,
- le 2-N-(β-hydroxyéthyl)-amino-5-β-γ-dihydroxypropyloxy-nitrobenzène,
- le 3-hydroxy-4-N-(β-hydroxyéthyl)-amino-nitrobenzène,
- le 4-amino-3-hydroxy-nitrobenzène,
- le 2,5-N,N'-(β-hydroxyéthyl)-amino-nitrobenzène,
- le 2-N-méthylamino-4-β-γ-dihydroxypropyloxy-nitrobenzène,
- le 2-N-(β-aminoéthyl)-amino-5-N,N-bis-(β-hydroxyéthyl)-amino-nitrobenzène,
- le 2-N-(β-aminoéthyl)-amino-4-méthoxy-nitrobenzène,
- le 2-N-(β-aminoéthyl)-amino-5-β-hydroxyéthyloxy-nitrobenzène,
- le 4'-hydroxy-2-nitro-diphénylamine,
- le 4-amino-2-nitro-diphénylamine,
- le 1-N-(β-uréidoéthyl)-amino-4-nitro-benzène,
- le 4-N-éthyl, N-(β-hydroxyéthyl)-amino-1-(β-hydroxyéthyl)-amino-2-nitro-benzène,
- le 4-N-(β-γ-dihydroxypropyl)-amino-3-nitro-trifluorométhylbenzène,
- le 4-N-(β-hydroxyéthyl)-amino-3-nitro-méthylbenzène,
- le 1-N-(β-hydroxyéthyl)-amino-4-N,N-bis-(β-hydroxyéthyl)-amino-2-nitro-benzène,
- le 2-amino-6-chloro-4-nitro-phénol,
- le 4-chloro-1,3-diamino-6-nitro-benzène,
- le 1-hydroxy-4-N-(γ-hydroxypropyl)-amino-3-nitro-benzène.

Parmi les colorants anthraquinoniques utilisables en association avec les colorants de formule (I) ou (II), on préfère utiliser les colorants connus sous les dénominations «Disperse Violet 4», «Disperse Blue 1», «Acid Violet 43», «Disperse Violet 1», «Disperse Red 11»_{,} «Acid Blue 62», «C.I.Mordant Red 3», dans le COLOR INDEX, 3ème édition, ainsi que le méthylsulfate du (4-hydroxyanthraquinonyl-1-aminopropyl)-méthylmorpholinium.

Parmi les nitropyridines utilisables en association avec les colorants de formule (I) ou (Il), on préfère utiliser :
- la 2,5-diamino-6-nitro-pyridine,
- la 5-amino-2-N-(β-hydroxyéthyl)-amino-6-nitro-pyridine,
- la 2-amino-5-N-(β-hydroxyéthyl)-amino-6-nitro-pyridine,
- la 5-amino-2-N-éthylamino-6-nitro-pyridine,
- la 2-N-éthylamino-5-N-(β-hydroxyéthyl)-amino-6-nitro-pyridine,
- la 2-N-méthylamino-5-N-(β-hydroxyéthyl)-amino-6-nitro-pyridine,

Parmi les colorants azoïques utilisables en association avec les colorants de formule (I) ou (II), on préfère utiliser ceux connus sous les dénominations «Disperse Yellow 3», «Basic Red 76», «Basic Brown 16», «Basic Yellow 57», «Acid Yellow 36», «Food Red 1», «Acid Orange 7», «Acid Red 88», «Food Yellow 3», «Acid Red 184», «Acid Orange 24», «Basic Brown 4», «Acid Red 35», «Disperse Red 17», «Disperse Black 5», dans le COLOR INDEX.

Parmi les colorants triarylméthaniques utilisables en association avec les colorants de formule (I) ou (II), on préfère utiliser ceux connus sous les dénominations «Basic Green 1», «Basic Violet 14», «Basic Violet 1», «Basic Violet 3», «Basic Blue 26», dans le COLOR INDEX.

Parmi les autres familles de colorants directs cités ci-avant et plus particulièrement utilisables en association avec les colorants de formule (I) ou (II), notamment parmi les aziniques, on peut citer : le «Basic Red 2», parmi les acridiniques, on peut citer : l'orangé d'acridine, dénommé «Basic Orange 14», dans le COLOR INDEX, et parmi les xanthéniques, on peut citer : la rhodamine B, dénommée «Basic Violet 10» dans le COLOR INDEX.

La proportion de tous ces autres colorants directs d'addition peut varier entre environ 0,05 et 10 % en poids par rapport au poids total de la composition tinctoriale.

Les colorants de formule formule (I) ou (Il) peuvent également être incorporés dans des compositions tinctoriales pour la coloration d'oxydation qui contiennent des bases d'oxydation et éventuellement des coupleurs, pour enrichir en reflets les nuances obtenues avec les colorants d'oxydation.

Le milieu approprié pour la teinture est de préférence un milieu aqueux constitué par de l'eau et/ou des solvants organiques acceptables sur le plan cosmétique, et plus particulièrement, des alcools tels que l'alcool éthylique, l'alcool isopropylique, l'alcool benzylique, et l'alcool phényléthylique, ou des glycols ou éthers de glycol tels que, par exemple, l'éthylèneglycol et ses éthers monométhylique, monoéthylique et monobutylique, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.
On peut également ajouter à la composition selon l'invention des amides gras tels que les mono- et di-éthanolamides des acides dérivés du coprah, de l'acide laurique ou de l'acide oléïque, à des concentrations comprises entre environ 0,05 et 10% en poids.
On peut encore ajouter à la composition selon l'invention des agents tensio-actifs bien connus de l'état de la technique et de type anionique, cationique, non-ionique, amphotère, zwittérionique ou leurs mélanges, de préférence en une proportion comprise entre environ 0,1 et 50% en poids et avantageusement entre environ 1 et 20% en poids par rapport au poids total de la composition. On peut également utiliser des agents épaississants dans une proportion allant d'environ 0,2 à 5%.
Ladite composition tinctoriale peut contenir en outre divers adjuvants usuels tels que des agents anti-oxydants, des parfums, des agents séquestrants, des agents dispersants, des agents de conditionnement du cheveu, des agents conservateurs, des agents opacifiants, ainsi que tout autre adjuvant utilisé habituellement en cosmétique.

La composition tinctoriale selon l'invention peut être formulée à pH acide, neutre ou alcalin, le pH pouvant varier par exemple de 4 à 11 et de préférence de 5 à 10, et pouvant être ajusté au moyen d'agents d'alcalinisation ou d'agents d'acidification antérieurement bien connus.

Comme agents alcalinisants, on peut citer l'ammoniaque, les carbonates alcalins, les alcanolamines, par exemple les mono- di- et tri- éthanolamines et leurs dérivés, les hydroxydes de sodium ou de potassium, et les composés de formule : 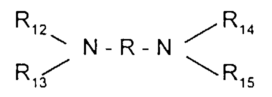 dans laquelle, R est un reste propylène éventuellement substitué par un groupement hydroxyle ou un radical alkyle en C₁-C₄ ; R₁₂ R₁₃, R₁₄ et R₁₅, simultanément ou indépendamment l'un de l'autre, représentent un atome d'hydrogène, un radical alkyle en C₁-C₄ ou hydroxyalkyle en C₁-C₄.
Les agents acidifiants sont classiquement des acides minéraux ou organiques comme par exemple les acides chlorhydrique, tartrique, citrique et phosphorique.

Lorsque la composition tinctoriale selon l'invention constitue une teinture éclaircissante, le pH de la composition (A) qui renferme au moins un colorant direct de formule (I) ou (Il) ainsi que celui de la composition (B) renfermant l'agent oxydant sont tels, qu'après mélange de la composition (A) avec la composition (B), le pH de la composition appliquée sur les fibres kératiniques humaines est supérieur à 7, et varie de préférence entre 8,5 et 11. Il est ajusté à la valeur choisie à l'aide d'agents alcalinisants et éventuellement acidifiants bien connus et par exemple décrits ci-avant.
L'agent oxydant est choisi de préférence parmi le peroxyde d'hydrogène, le peroxyde d'urée, les bromates de métaux alcalins, les persels tels que les perborates et les persulfates. L'utilisation du peroxyde d'hydrogène est particulièrement préférée.
La composition oxydante (B) est de préférence constituée par une solution d'eau oxygénée dont le titre peut varier, plus particulièrement, d'environ 5 à 40 volumes.
L'agent alcalinisant est de préférence choisi parmi les alcanolamines quand un éclaircissement modéré est recherché, et il est plus particulièrement représenté par l'ammoniaque lorsqu'un éclaircissement plus important est souhaité.
La composition appliquée sur les cheveux peut se présenter sous des formes diverses, telles que sous forme de liquide, de crème, de gel ou sous toute autre forme appropriée pour réaliser une teinture des fibres kératiniques. En particulier, elle peut être conditionnée sous pression en flacon aérosol en présence d'un agent propulseur et former une mousse.

Un autre objet de la présente invention porte sur un procédé de teinture des fibres kératiniques, en particulier des fibres kératiniques humaines telles que les cheveux, par coloration directe, consistant à laisser agir une composition tinctoriale renfermant au moins un colorant direct de formule (I) ou (II) sur les fibres kératiniques sèches ou humides. On peut utiliser la composition selon l'invention en tant que composition non rincée, c'est-à-dire qu'après application de la composition sur les fibres, on sèche sans rinçage intermédiaire.
Dans les autres modes d'application, on laisse agir la composition sur les fibres pendant un temps de pose variant entre 3 et 60 minutes environ, de préférence entre 5 et 45 minutes environ, on rince, éventuellement on lave, puis on rince à nouveau, et on sèche.

L'invention a également pour objet un procédé de teinture des fibres kératiniques, en particulier des fibres kératiniques humaines telles que les cheveux, consistant à appliquer sur ces fibres au moins une composition (A) contenant, dans un milieu approprié pour la teinture, au moins un colorant direct de formule (I) ou (II), l'éclaircissement étant assuré, à pH supérieur à 7, à l'aide d'un agent oxydant qui est mélangé juste au moment de l'emploi à la composition (A), ou qui est présent dans une composition (B) appliquée simultanément.

L'invention a également pour objet des dispositifs de teinture ou «kits» à plusieurs compartiments, dont le premier compartiment contient au moins un colorant direct de formule (I) ou (II), ainsi qu'un agent alcalinisant, et le deuxième compartiment, un agent oxydant. Une autre alternative de «kit» se compose d'un premier compartiment comprenant au moins un colorant de formule (I) ou (II), un deuxième compartiment comprenant un agent alcalinisant et un troisième compartiment comprenant un agent oxydant.

Un autre objet de la présente invention concerne les nouvelles 4,5-diiminopyrazolines de formule (I) ou leur forme tautomère 4-ylidèneamino-5-amino-pyrazoles de formule (Il) ci-avant définies, sous réserve que,
(i) lorsque R₁ désigne phényle, que R₂ désigne méthyle, et que R₃ désigne hydrogène, alors A est différent de 4-diméthylaminophényle et de 4-diéthylaminophényle,
(ii) lorsque R₁, R₂, et R₃ désignent hydrogène, alors A est différent des radicaux para-(2-méthyl-5-aminophénol) et para-(1,3-diaminobenzène).

Les composés nouveaux de formule (I) peuvent être obtenus suivant un procédé de préparation classiquement connu, selon lequel on fait réagir un 5-aminopyrazole (1) sur un composé benzénique (2), en présence d'un oxydant tel que le ferricyanure de potassium, et de préférence à raison de 1 à 3 équivalents molaires, pour obtenir une 4,5-diiminopyrazoline de formule (I), comme l'indique le schéma suivant : 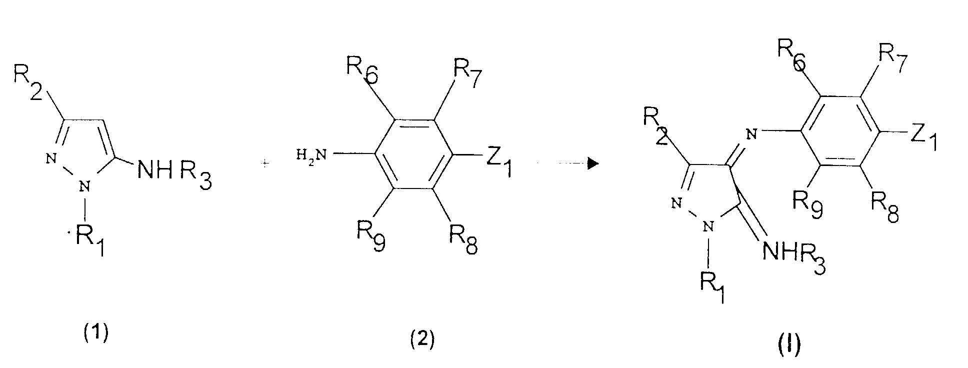 La réaction est conduite à une température comprise entre 5 et 50°C dans un mélange eau/pyridine, tamponné à pH 8 à l'aide d'un tampon phosphate ou carbonate, la teneur en pyridine étant de préférence inférieure à 30%.

Les composés nouveaux de formule (Il) peuvent être obtenus selon un procédé de préparation, lui-même constitutif d'un autre objet de la présente invention, qui consiste à faire réagir un 4,5-diaminopyrazole (3) avec un composé benzénique (4) de façon à obtenir le 4,5-diaminopyrazole de formule (Il), selon le schéma suivant : 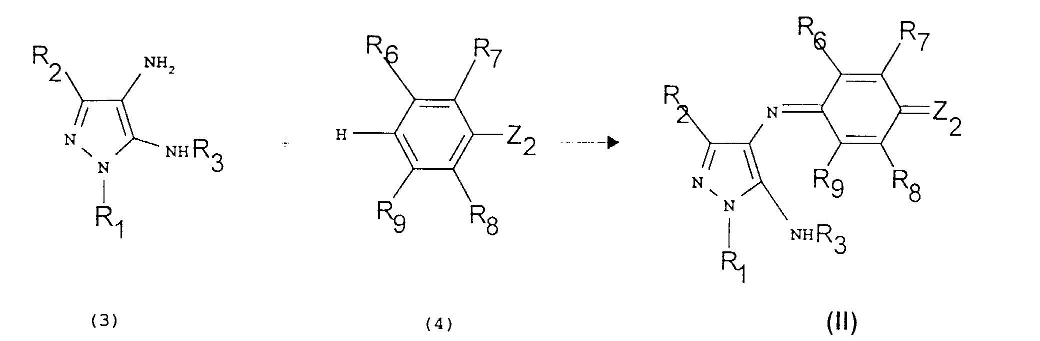 La réaction est conduite à une température comprise entre 10°C et 60°C, en milieu hydroalcoolique, l'alcool étant choisi de préférence parmi les alcools inférieurs tels que le méthanol, l'éthanol ou l'isopropanol.
Les composés (3) et (4) peuvent être sous forme de bases libres ou de sels organiques ou minéraux. Lorsqu'ils sont sous forme de bases libres, le pH de la réaction est un pH spontané. Lorsque l'un des composés (3) ou (4) ou les deux composés (3) et (4) sont sous forme de sels, on utilise une base en quantité stoechiométrique, et de préférence un hydroxyde de sodium ou de potassium ou de lithium.

Le composé (4) est de préférence un composé de formule : 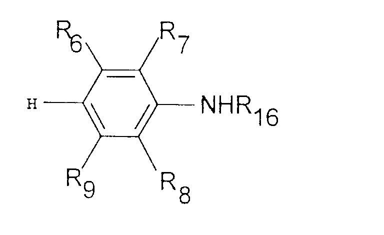 dans laquelle R₆, R₇, R₈, R₉ prennent les mêmes significations que celles ci-avant définies et R₁₆ désigne hydrogène ou alkyle linéaire ou ramifié en C₁-C₄, ou un composé de formule : 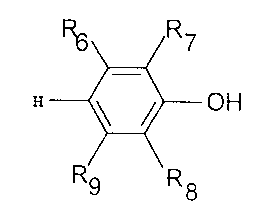 dans laquelle R₆, R₇, R₈, R₉ prennent les mêmes significations que ci-avant.

Des exemples concrets illustrant l'invention vont maintenant être donnés.

### EXEMPLES DE PREPARATION

### EXEMPLE 1 : Préparation de la 5-amino-2-méthyl-N-(5-amino-pyrazol-4-yl)-p-quinone-monoimine (IIa).

A une solution de 43 g (0,25 mole) de dichlorhydrate de 4,5-diaminopyrazole dans 500 cm³ d'eau, on a ajouté à température ambiante, 31 g (0,25 mole) de 5-amino-2-méthyl-phénol dissous dans 400 cm³ d'éthanol. On a ensuite ajouté à 25°C, 32 g (0,5 mole) d'hydroxyde de potassium dissous dans 80 cm³ d'eau. Après avoir fait barboter de l'air pendant 48 h sous agitation, on a refroidi le milieu réactionnel à 0°C, essoré le précipité, lavé celui-ci par 100 cm³ d'eau et séché sous 30 mm Hg à 40°C.
On a obtenu 27 g de 5-amino-2-méthyl-N-(5-amino-pyrazol-4-yl)-p-quinone-monoimine (lla), sous forme d'un solide rouge-marron dont le point de fusion était compris entre 250°C et 251°C.
L'analyse élémentaire pour **C**₁₀ **H**₁₁ **N**₅ **O**, 1 H₂O était:

| % | **C** | **H** | **N** | **O** |
|---|---|---|---|---|
| théorie | 51,05 | 5,57 | 29,77 | 13,60 |
| trouvé | 51,10 | 5,60 | 29,75 | 13,75 |

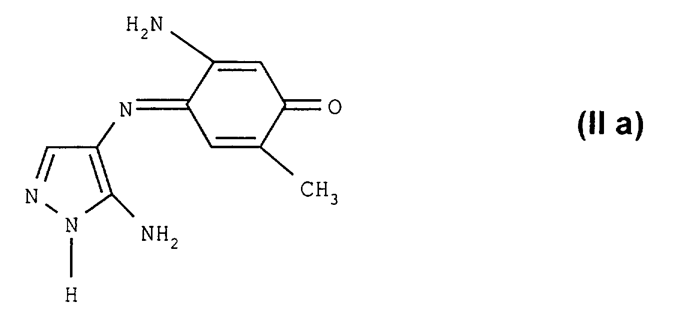

II peut se transformer, lors de sa mise en oeuvre dans les compositions de teinture, dans sa forme tautomère correspondante 5-imino-4[(2'-amino-4'-hydroxy-5'-méthyl)-phényl]-imino-pyrazoline, suivant un équilibre tautomérique.

### EXEMPLE 2 : Préparation de la 5-amino-2-méthyl-N-(5-amino-1-méthyl-pyrazol-4-yl)-p-quinone-monoimine (IIb).

A une solution de 46 g (0,25 mole) de dichlorhydrate de 4,5-diamino-1-méthyl-pyrazole dans 500 cm³ d'eau, on a ajouté à température ambiante, 31 g (0,25 mole) de 5-amino-2-méthyl-phénol dissous dans 400 cm³ d'éthanol. On a ensuite ajouté à 25°C, 32 g (0,5 mole) d'hydroxyde de potassium dissous dans 80 cm³ d'eau. Après avoir fait barboter de l'air pendant 48 h sous agitation, on a refroidi le milieu réactionnel à 0°C, essoré le précipité, lavé celui-ci par 100 cm³ d'eau et séché sous vide à 40°C.
On a obtenu 56 g de 5-amino-2-méthyl-N-(5-amino-1-méthyl-pyrazol-4-yl)-p-quinone-monoimine (IIb), sous forme d'un solide marron-rouge dont le point de fusion était de 160°C.
L'analyse élémentaire pour **C**₁₁ **H**₁₃ **N**₅ **O**, 1 H₂O était :

| % | **C** | **H** | **N** | **O** |
|---|---|---|---|---|
| théorie | 53,00 | 6,06 | 28,10 | 12,84 |
| trouvé | 53,15 | 6,10 | 28,28 | 12,75 |

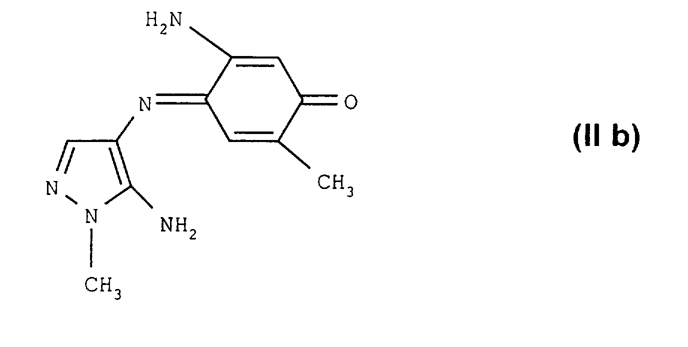

II peut se transformer, lors de sa mise en oeuvre dans les compositions de teinture, dans sa forme tautomère correspondante 5-imino-4-[(2'-amino-4'-hydroxy-5'-méthyl)-phényl]-imino-1-méthyl-pyrazoline, suivant un équilibre tautomérique.

### EXEMPLE 3 : Préparation de la 5-amino-2-méthyl-N-(5-amine-1,3-diméthylpyrazol-4-yl)-p-quinone-monoimine (IIc).

A une solution de 50 g (0,25 mole) de dichlorhydrate de 4,5-diamino-1,3-diméthylpyrazole dans 500 cm³ d'eau, on a ajouté à température ambiante, 31 g (0,25 mole) de 5-amino-2-méthyl-phénol dissous dans 400 cm³ d'éthanol. On a ensuite ajouté à 25°C, 32 g (0,5 mole) d'hydroxyde de potassium dissous dans 80 cm³ d'eau. Après avoir fait barboter de l'air pendant 48 h sous agitation, on a refroidi le milieu réactionnel à 0°C, essoré le précipité, lavé celui-ci par 100 cm³ d'eau et séché sous vide à 40°C.
On a obtenu 30 g de 5-amino-2-méthyl-N-(5-amino-1,3-diméthyl-pyrazol-4-yl)-p-quinone-monoimine (IIc), sous forme d'un solide marron dont le point de fusion était compris entre 245°C et 246°C.
L'analyse élémentaire pour **C**₁₂ **H**₁₅ **N**₅ **O**, 1 H₂O était :

| % | **C** | **H** | **N** | **O** |
|---|---|---|---|---|
| théorie | 54,74 | 6,51 | 26,60 | 12,15 |
| trouvé | 54,45 | 6,52 | 26,60 | 12,00 |

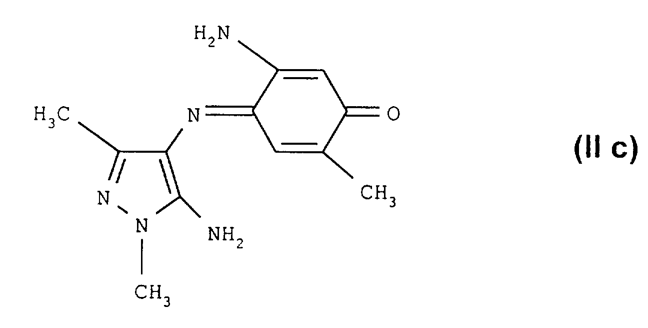

Il peut se transformer, lors de sa mise en oeuvre dans les compositions de teinture, dans sa forme tautomère correspondante 5-imino-4-[(2'-amino-4'-hydroxy-5'-méthyl)-phényl]-imino-1,3-diméthyl-pyrazoline, suivant un équilibre tautomérique.

### EXEMPLES DE COMPOSITIONS TINCTORIALES

### EXEMPLE 4 :

On a préparé la composition de teinture suivante :

| | |
|---|---|
| Alcool oléïque polyglycérolé à 2 moles de glycérol | 4,0 g |
| Alcool oléïque polyglycérolé à 4 moles de glycérol (à 78% MA) | 5,69g MA* |
| Acide oléïque | 3,0 g |
| Amine oléïque oxyéthylénée à 2 moles d'oxyde d'éthylène | 7,0 g |
| Laurylamino succinamate de diéthylaminopropyle, sel de sodium (à 55% MA | 3,0 g MA |
| Alcool oléïque | 5,0 g |
| Diéthanolamide d'acide oléïque | 12,0 g |
| Propylène glycol | 3,5 g |
| Alcool éthylique | 7,0 g |
| Dipropylène glycol | 0,5 g |
| Monométhyléther de propylène glycol | 9,0 g |
| Parfum, conservateur, | q.s. |
| Monoéthanolamine q.s | pH 9.8 |
| 2-amino-5-N-(β-hydroxyéthyl)-amino-nitrobenzène | 0,15g |
| Composé préparé à l'exemple 3 | 0,5 g |
| Eau déminéralisée q.s.p | 100 g |

| | |
|---|---|
| *Matière Active | |

On a mélangé la composition ci-dessus avec 2 fois son poids en eau oxygénée titrant 9 volumes.
On a appliqué le mélange obtenu sur des mèches de cheveux gris naturels à 90% de blancs et on a laissé poser pendant 15 minutes. Après rinçage à l'eau courante, lavage avec un shampooing conventionnel et séchage, les cheveux ont été teints dans une nuance irisé-cendré.

### EXEMPLE 5:

On a préparé la composition de teinture suivante :

| | |
|---|---|
| 2-N-méthylamino-4-β,γ-dihydroxypropyloxy-nitrobenzène | 0,2 g |
| Composé préparé à l'exemple 2 | 0,4g |
| Monométhyléther de propylène glycol | 10 g |
| Lauryléther sulfate de sodium oxyéthyléné par 2,2 moles d'oxyde d'éthylène (à 28% MA) | 5,6g MA* |
| 2-amino-2-méthyl-1-propanol q.s | pH 7 |
| Eau déminéralisée q.s.p | 100 g |

| | |
|---|---|
| *Matière Active | |

On a appliqué la composition ci-dessus sur des mèches de cheveux gris naturels ou permanentés à 90% de blancs et on a laissé poser pendant 30 minutes. Après rinçage à l'eau courante et séchage, les cheveux ont été teints dans une nuance cuivré-doré.

### EXEMPLE 6 :

On a préparé la composition de teinture suivante :

| | |
|---|---|
| Composé préparé à l'exemple 3 | 0,5g |
| Ethanol | 10,0g |
| Monoéthanolamine q.s | pH 10 |
| Eau q.s.p | 100 g |

On a appliqué la composition ci-dessus sur des mèches de cheveux gris permanentés à 90% de blancs et on a laissé poser pendant 30 minutes. Après un rinçage à l'eau courante, un shampooing conventionnel, rinçage à nouveau et séchage, les cheveux ont été teints dans une nuance rose-violacé.

### EXEMPLE 7 :

On a préparé la composition de teinture suivante :

| | |
|---|---|
| Composé préparé à l'exemple 3 | 1,0g |
| Ethanol | 10 g |
| Solution aqueuse d'NH3 à 20% q.s | pH 10,5 |
| eau q.s.p | 100 g |

On a mélangé la composition ci-dessus avec son poids en eau oxygénée titrant 20 volumes.
On a appliqué le mélange obtenu sur des mèches de cheveux gris permanentés à 90% de blancs et on a laissé poser pendant 30 minutes. Après rinçage à l'eau courante, lavage avec un shampooing conventionnel, rinçage et séchage, les cheveux ont été teints dans une nuance rose fuchsia.

## Revendications

1. Composition de teinture pour fibres kératiniques, en particulier pour fibres kératiniques humaines, telles que les cheveux, caractérisée par le fait qu'elle contient, dans un milieu approprié pour la teinture, au moins un composé 4,5-diiminopyrazoline de formule (I) : 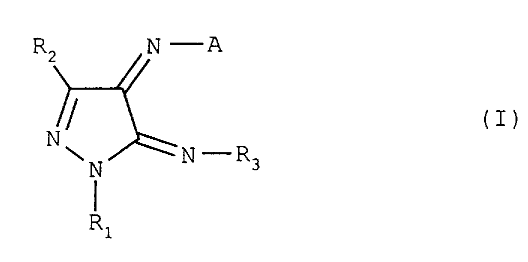 et/ou sa forme tautomère 4-ylidèneamino-5-amino-pyrazole de formule (II) : 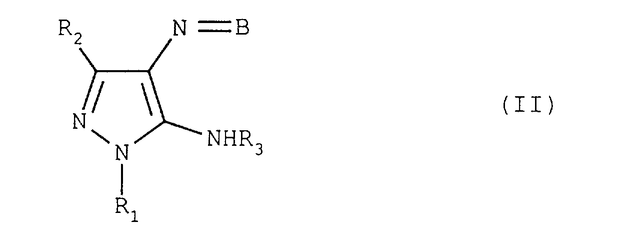 formules dans lesquelles,
**R**_{**1**} et **R**_{**3**}, identiques ou différents, représentent un atome d'hydrogène, un radical alkyle en C₁-C₆, ou hydroxyalkyle en C₂-C₄, ou aminoalkyle en C₂-C₄, un radical phényle ou phényle substitué par un atome d'halogène ou par un radical alkyle en C₁-C₄, ou alcoxy en C₁-C₄, ou nitro, ou trifluorométhyle, ou amino, ou alkylamino en C₁-C₄, un radical benzyle ou benzyle substitué par un atome d'halogène ou par un radical alkyle en C₁-C₄, ou alcoxy en C₁-C₄, ou méthylènedioxy ou amino,
un radical : 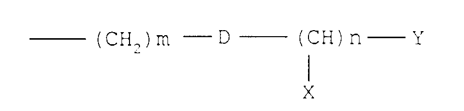 dans lequel m et n désignent un nombre entier allant de 1 à 3, inclusivement,
D désigne oxygène ou -NH-,
X désigne hydrogène ou CH₃,
Y désigne CH₃ ou OR₄, R₄ désignant hydrogène, méthyle, ou éthyle,
et R₃ peut également désigner un radical amino,
**R**_{**2**} représente un atome d'hydrogène, un radical alkyle en C₁-C₆, ou hydroxyalkyle en C₁-C₄, ou aminoalkyle en C₁-C₄, ou alkylthio en C₁-C₂, ou alcoxy en C₁-C₅, ou trifluorométhyle, ou cyano, ou carboxyle, un radical phényle ou phényle substitué par un atome d'halogène ou par un radical alkyle en C₁-C₄, ou alcoxy en C₁-C₄, ou nitro, ou trifluorométhyle, ou amino, ou alkylamino en C₁-C₄, un radical benzyle ou benzyle substitué par un atome d'halogène ou par un radical alkyle en C₁-C₄, ou alcoxy en C₁-C₄, ou nitro, ou trifluorométhyle, ou amino, ou alkylamino en C₁-C₄, un hétérocycle choisi parmi le thiophène, le furane ou la pyridine, un radical :
―(CH₂)p―O―(CH₂)q―OR₅
dans lequel p et q, identiques ou différents, sont des nombres entiers allant de 1 à 3 inclusivement, et R₅ désigne hydrogène ou méthyle,
**A** représente, dans la formule (I), le radical : 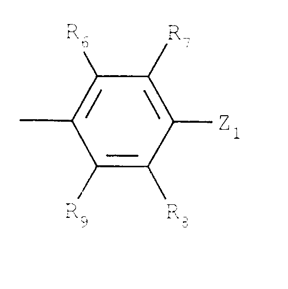 et,
**B** représente dans la formule (II), le radical : 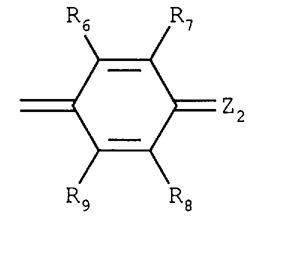 radicaux dans lesquels,
**Z**_{**1**} représente un radical hydroxy ou alcoxy en C₁-C₄, ou amino, ou acétamido, ou alkylamino en C₁-C₄, ou hydroxyalkylamino en C₁-C₄, ou aminoalkylamino en C₁-C₄, un radical N R₁₀ R₁₁ dans lequel R₁₀ et R₁₁, identiques ou différents, désignent un radical alkyle en C₁-C₄, ou hydroxyalkyle en C₂-C₄ ou aminoalkyle en C₂-C₄.
**R**_{**6**}**, R**_{**7**}**, R**_{**8**}**, R**_{**9**}**,** identiques ou différents, représentent, un atome d'hydrogène, un radical alkyle en C₁-C₄, ou alcoxy en C₁-C₄, ou alkylthio en C₁-C₄, un radical amino, alkylamino en C₁-C₄, ou carbamoylméthylamino, ou hydroxyalkyle en C₂-C₄, ou monohydroxyalcoxy en C₂-C₄, ou dihydroxyalcoxy en C₂-C₄, un radical carboxyalcoxy en C₁-C₂, un atome de chlore, ou de brome, ou de fluor, un radical trifluoroalkyle en C₁-C₂,
un radical : 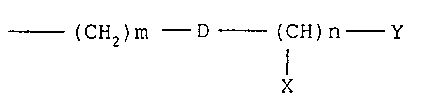 dans lequel, m, n, D, X, Y, ont les mêmes significations que ci-dessus, ou un radical N R₁₀ R₁₁ avec, pour R₁₀ et R₁₁ les mêmes significations que ci-dessus,
**Z**_{**2**} représente O, ou NR, avec R désignant H, ou acétyle, ou alkyle en C₁-C₄, ou hydroxyalkyle en C₁-C₄, ou aminoalkyle en C₁-C₄,
étant entendu que tous les radicaux alkyle ou alcoxy des groupements ci-avant définis peuvent être linéaires ou ramifiés, et sous réserve que,
(i) lorsque R₁ désigne phényle, que R₂ désigne méthyle, et que R₃ désigne hydrogène, A est différent de 4-diméthylaminophényle et de 4-diéthylaminophényle,
(ii) lorsque R₁, R₂, et R₃ désignent hydrogène, A est différent des radicaux 2-amino-4-hydroxy-5-méthylphényle et 2,4-diaminophényle.

2. Composition selon la revendication 1, caractérisée par le fait qu'elle contient un composé choisi dans le groupe constitué par :
- le 5-imino-4-[(2'-amino-4'-hydroxy-5'-méthyl)-phényl]-imino-1,3-diméthyl-pyrazoline,
- le 5-imino-4-[(2'-amino-4'-hydroxy-5'-méthyl)-phényl]-imino-1-méthyl-pyrazoline,
- le 5-imino-4-[(2'-amino-4'-hydroxy-5'-méthyl)-phényl]-imino-3-méthyl-pyrazoline,
- le 5-imino-4-[(2'-amino-4'-hydroxy)-phényl]-imino-1,3-diméthyl-pyrazoline,
- le 5-imino-4-[(2'-amino-4'-hydroxy)-phényl]-imino-1-méthyl-pyrazoline,
- le 5-imino-4-[(2'-amino-4'-hydroxy)-phényl]-imino-pyrazoline,
- le 5-imino-4-[(2'-amino-4'-hydroxy)-phényl]-imino-3-méthyl-pyrazoline,
- le 1-éthyl-5-imino-4-[(2'-amino-4'-hydroxy-5'-méthyl)-phényl]-imino-3-méthyl-pyrazoline,
- le 1-éthyl-5-imino-4-[(2'-amino-4'-hydroxy-5'-méthyl)-phényl]-imino-pyrazoline,
- le 1-éthyl-5-imino-4-[(2'-amino-4'-hydroxy)-phényl]-imino-3-méthyl-pyrazoline,
- le 1-éthyl-5-imino-4-[(2'-amino-4'-hydroxy)-phényl]-imino-pyrazoline,
- le 1-isopropyl-5-imino-4-[(2'-amino-4'-hydroxy-5'-méthyl)-phényl]-imino-pyrazoline,
- le 1-isopropyl-5-imino-4-[(2'-amino-4'-hydroxy-5'-méthyl)-phényl]-imino-3-méthyl-pyrazoline,
- le 1-isopropyl-5-imino-4-[(2'-amino-4'-hydroxy)-phényl]-imino-pyrazoline,
- le 1-isopropyl-5-imino-4-[(2'-amino-4'-hydroxy)-phényl]-imino-3-méthyl-pyrazoline,
- le 1-(β-hydroxyéthyl)-5-imino-4-[(2'-amino-4'-hydroxy)-phényl]-imino-pyrazoline,
- le 1-(β-hydroxyéthyl)-5-imino-4-[(2'-amino-4'-hydroxy)-phényl]-imino-3-méthyl-pyrazoline,
- le 1-(β-hydroxyéthyl)-5-imino-4-[(2'-amino-4'-hydroxy-5'-méthyl)-phényl]-imino-pyrazoline,
- le 1-(β-hydroxyéthyl)-5-imino-4-[(2'-amino-4'-hydroxy-5'-méthyl)-phényl]-imino-3-méthyl-pyrazoline,
- le 5-imino-4-[(2'-amino-4'-hydroxy-5'-méthyl)-phényl]-imino-3-trifluorométhyl-pyrazoline,
- le 1-méthyl-5-imino-4-[(2'-amino-4'-hydroxy-5'-méthyl)-phényl]-imino-3-trifluorométhyl-pyrazoline,
- le 1-méthyl-5-imino-4-[(2'-amino-4'-hydroxy)-phényl]-imino-3-trifluorométhyl-pyrazoline,
- le 5-imino-4-[(2'-amino-4'-hydroxy)-phényl]-imino-3-trifluorométhyl-pyrazoline,
- le 1-éthyl-5-imino-4-[(2'-amino-4'-hydroxy-5'-méthyl)-phényl]-imino-3-trifluorométhyl-pyrazoline,
- le 1-éthyl-5-imino-4-[(2'-amino-4'-hydroxy)-phényl]-imino-3-trifluorométhyl-pyrazoline,
- le 1-isopropyl-5-imino-4-[(2'-amino-4'-hydroxy-5'-méthyl)-phényl]-imino-3-trifluorométhyl-pyrazoline,
- le 1-isopropyl-5-imino-4-[(2'-amino-4'-hydroxy)-phényl]-imino-3-trifluorométhyl-pyrazoline,
- le 1-(β-hydroxyéthyl)-5-imino-4-[(2'-amino-4'-hydroxy)-phényl]-imino-3-trifluorométhyl-pyrazoline,
- le 1-(β-hydroxyéthyl)-5-imino-4-[(2'-amino-4'-hydroxy-5'-méthyl)-phényl]-imino-3-trifluorométhyl-pyrazoline,
et/ou leur forme tautomère 4-ylidèneamino-5-amino-pyrazole correspondante, lorsque la tautomérie est possible.

3. Composition selon l'une quelconque des revendications précédentes, caractérisée par le fait qu'elle a un pH compris entre 4 et 11.

4. Utilisation des composés de formule (I) ou (Il) tels que définis à la revendication 1, à titre de colorant direct dans des, ou pour la préparation de, compositions tinctoriales des fibres kératiniques.

5. Utilisation des composés de formule (I) ou (Il) tels que définis à la revendication 1, à titre de colorant direct dans des, ou pour la préparation de, compositions destinées à la teinture directe éclaircissante des fibres kératiniques.

6. Composition de teinture directe pour fibres kératiniques, en particulier pour fibres kératiniques humaines telles que les cheveux, caractérisée par le fait qu'elle contient, dans un milieu approprié pour la teinture, au moins un colorant direct de formule (I) ou (Il) tel que défini à la revendication 1 ou 2.

7. Composition de teinture directe éclaircisssante pour fibres kératiniques, en particulier pour fibres kératiniques humaines telles que les cheveux, caractérisée par le fait qu'elle contient, dans un milieu approprié pour la teinture, au moins un colorant direct de formule (I) ou (Il) tel que défini à la revendication 1 ou 2.

8. Composition selon la revendication 7, prête à l'emploi, caractérisée par le fait qu'elle contient en plus, un agent oxydant, et un agent alcalinisant en quantité suffisante pour ajuster le pH final à une valeur supérieure à 7.

9. Composition selon l'une quelconque des revendications précédentes, caractérisée par le fait que le ou les composés de formule (I) ou (Il) sont présents dans une concentration allant de 0,001 à 5% en poids par rapport au poids total de la composition avant leur application sur les fibres.

10. Composition selon l'une quelconque des revendications précédentes, caractérisée par le fait que le ou les composés de formule (I) ou (Il) sont présents dans une concentration allant de 0,01 à 3% en poids par rapport au poids total de la composition avant leur application sur les fibres.

11. Composition selon la revendication 8, caractérisée par le fait que l'agent oxydant est constitué par de l'eau oxygénée.

12. Composition selon la revendication 8, caractérisée par le fait que l'agent alcalinisant est choisi parmi l'ammoniaque ou une alcanolamine.

13. Composition selon l'une quelconque des revendications 8 à 12, caractérisée par le fait que l'agent alcalinisant est présent en une quantité suffisante pour ajuster le pH final entre les valeurs allant de 8,5 à 11.

14. Composition selon l'une quelconque des revendications précédentes, caractérisée par le fait qu'elle contient d'autres colorants directs choisis parmi des colorants nitrés benzéniques comme les nitrophénylènediamines, les nitrodiphénylamines, les nitroanilines, les éthers de phénols nitrés ou les nitrophénols, des nitropyridines, des colorants anthraquinoniques, mono- ou diazoïques, triarylméthaniques, aziniques, acridiniques, xanthéniques, ou des colorants métallifères.

15. Composition selon l'une quelconque des revendications précédentes, caractérisée par le fait que le milieu approprié pour la teinture est un milieu aqueux constitué par de l'eau et/ou des solvants organiques choisis parmi les alcools, les glycols et les éthers de glycol, dans des proportions comprises entre 0,5 et 20% en poids par rapport au poids total de la composition.

16. Procédé de teinture des fibres kératiniques et en particulier des fibres kératiniques humaines telles que les cheveux, par coloration directe, caractérisé par le fait qu'on applique la composition tinctoriale définie selon les revendications 1 à 3, 6, 9, 10, 14 et 15, sur les fibres kératiniques sèches ou humides, et qu'on sèche ces fibres sans rinçage intermédiaire.

17. Procédé de teinture des fibres kératiniques et en particulier des fibres kératiniques humaines telles que les cheveux, par coloration directe, caractérisé par le fait qu'on applique la composition tinctoriale définie selon l'une quelconque des revendications 1 à 3, 6, 9, 10, 14 et 15, sur les fibres kératiniques sèches ou humides, et qu'après avoir laissé agir la composition pendant 3 à 60 minutes environ, on rince les fibres, on les lave éventuellement, on les rince à nouveau puis on les sèche.

18. Procédé de teinture des fibres kératiniques et en particulier des fibres kératiniques humaines telles que les cheveux, par coloration directe éclaircissante, caractérisé par le fait qu'on applique la composition tinctoriale (A) définie selon l'une quelconque des revendications 1, 2, et 7 à 15, en provoquant un éclaircissement desdites fibres à pH supérieur à 7, à l'aide d'un agent oxydant qui est ajouté juste au moment de l'emploi à ladite composition de teinture ou qui est présent dans une composition (B) appliquée simultanément.

19. Dispositifs à plusieurs compartiments ou «kits» pour la teinture éclaircissante des fibres kératiniques humaines telles que les cheveux, caractérisés par le fait qu'ils comportent au moins deux compartiments, dont l'un d'eux renferme une composition (A) telle que définie à l'une quelconque des revendications 1, 2, et 7 à 15, et un autre une composition (B) comprenant un agent oxydant dans un milieu approprié pour la teinture.

20. 4,5-diiminopyrazolines de formules (I) ou (II) définies selon la revendication 1, sous réserve que,
(i) lorsque R₁ désigne phényle, que R₂ désigne méthyle, et que R₃ désigne hydrogène, A est différent de 4-diméthylaminophényle et de 4-diéthylaminophényle,
(ii) lorsque R₁, R₂, et R₃ désignent hydrogène, A est différent des radicaux 2-amino-4-hydroxy-5-méthylphényle et 2,4-diaminophényle.

21. Procédé de préparation d'un composé de formule (Il) telle que définie dans la revendication 20, caractérisé par le fait qu'il consiste à faire réagir, en milieu hydroalcoolique et à une température comprise entre 10 et 60°C, un 4,5-diaminopyrazole de formule (3) : 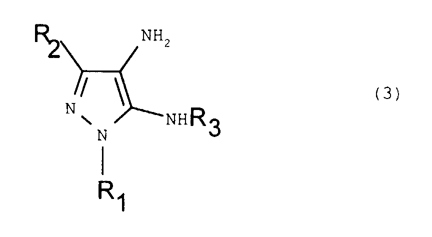 sur un composé de formule (4) :

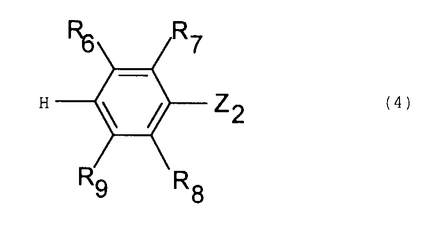

22. Procédé selon la revendication 21, caractérisé par le fait que le composé de formule (4) est un composé de formule : 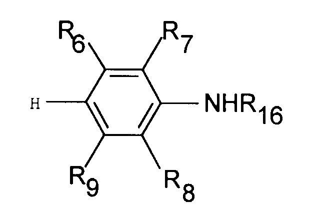 dans laquelle,
R₆, R₇, R₈, R₉ ont les mêmes significations que les radicaux R₆ à R₉ désignés ci-avant dans la revendication 20, et R₁₆ désigne hydrogène ou alkyle linéaire ou ramifié en C₁-C₄.

23. Procédé selon la revendication 21, caractérisé par le fait que le composé de formule (4) est un composé de formule :

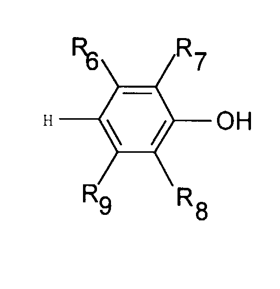

24. 5-amino-2-méthyl-N-(5-amino-1-méthyl-pyrazol-4-yl)-p-quinone-monoimine ainsi que sa forme tautomère 5-imino-4-[(2'-amino-4'-hydroxy-5'-méthyl)-phényl]-imino-1-méthyl-pyrazoline, selon la revendication 20.

25. 5-amino-2-méthyl-N-(5-amino-1,3-diméthylpyrazol-4-yl)-p-quinone-mono-imine ainsi que sa forme tautomère 5-imino-4-[(2'-amino-4'-hydroxy-5'-méthyl)-phényl]-imino-1,3-diméthyl-pyrazoline, selon la revendication 20.

## Patentansprüche

1. Färbemittelzusammensetzung für menschliche Keratinfasern, wie z.B. das Haar, dadurch gekennzeichnet, daß sie in einem für die Färbung geeigneten Medium mindestens eine 4,5-Diiminopyrazolin-Verbindung der Formel (I) 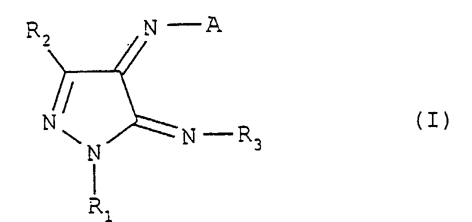 und/oder ihre tautomere 4-Ylidenamino-5-aminopyrazol-Form der Formel (II) 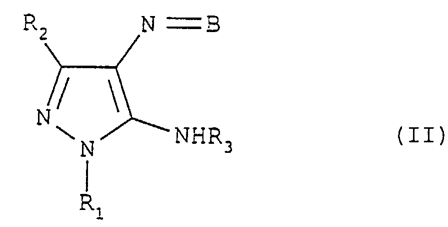 enthält, in denen bedeuten:
- R₁ und R₃, die gleich oder verschieden sind, Wasserstoff, C₁-C₆-Alkyl, C₂-C₄-Hydroxyalkyl, C₂-C₄-Aminoalkyl, Phenyl, Phenyl, das mit einem Halogenatom, C₁-C₄-Alkyl, C₁-C₄-Alkoxy, Nitro, Trifluormethyl, Amino, C₁-C₄-Alkylamino, Benzyl oder Benzyl substituiert ist, das mit einem Halogenatom, C₁-C₄-Alkyl, C₁-C₄-Alkoxy, Methylendioxy oder Amino substituiert ist,
eine Gruppe 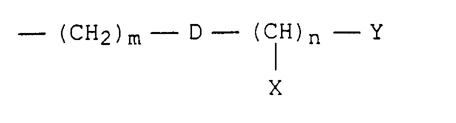 worin m und n eine ganze Zahl von 1 bis 3 einschließlich 1 und 3 darstellen, D Sauerstoff oder -NH-, X Wasserstoff oder -CH₃, Y -CH₃ oder -OR₄ darstellt, worin R₄ Wasserstoff, Methyl oder Ethyl bedeutet,
wobei R₃ außerdem eine Aminogruppe bedeutet,
- R₂ Wasserstoff, C₁-C₆-Alkyl, C₁-C₄-Hydroxyalkyl, C₁-C₄-Aminoalkyl, C₁-C₂-Alkylthio, C₁-C₅-Alkoxy, Trifluormethyl, Cyano, Carboxy, Phenyl oder Phenyl, das mit einem Halogenatom, C₁-C₄-Alkyl, C₁-C₄-Alkoxy, Nitro, Trifluormethyl, Amino, C₁-C₄-Alkylamino substituiert ist, Benzyl oder Benzyl, das mit einem Halogenatom, C₁-C₄-Alkyl, C₁-C₄-Alkoxy, Nitro, Trifluormethyl, Amino, C₁-C₄-Alkylamino substituiert ist, einen Heterocyclus, der unter Thiophen, Furan und Pyridin ausgewählt ist, eine Gruppe
―(CH₂)ₚ―O―(CH)_{q}―OR₅
worin p und q, die gleich oder verschieden sind, ganze Zahl von 1 bis 3 einschließlich 1 und 3 darstellen und R₅ Wasserstoff oder Methyl bedeutet,
- A in der Formel (I) die Gruppe 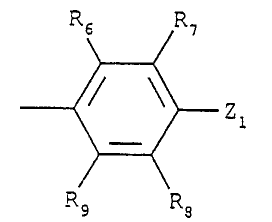 und
- B in der Formel (II) die Gruppe 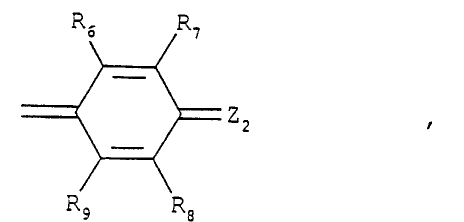 in denen bedeuten:
- Z₁ Hydroxy, C₁-C₄-Alkoxy, Amino, Acetamido, C₁-C₄-Alkylamino, C₁-C₄-Hydroxyalkylamino, C₁-C₄-Aminoalkyl-amino oder NR₁₀R₁₁, worin R₁₀ und R₁₁, die gleich oder verschieden sind, C₁-C₄-Alkyl, C₂-C₄-Hydroxyalkyl oder C₂-C₄-Aminoalkyl darstellen,
- R₆, R₇, R₈, R₉, die gleich oder verschieden sind, Wasserstoff, C₁-C₄-Alkyl, C₁-C₄-Alkoxy, C₁-C₄-Alkylthio, Amino, C₁-C₄-Alkylamino, Carbamoylmethylamino, C₂-C₄-Hydroxyalkyl, C₂-C₄-Monohydroxyalkoxy, C₂-C₄-Dihydroxyalkoxy, C₁-C₂-Carboxyalkoxy, Chlor, Brom, Fluor, C₁-C₂-Trifluoralkyl,
eine Gruppe 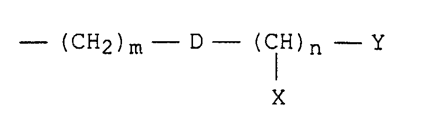 worin m, n, D, X, Y dasselbe wie weiter oben bedeuten, oder NR₁₀R₁₁, worin R₁₀ und R₁₁ dasselbe wie weiter oben bedeuten,
- Z₂ -O-, NR, worin R Wasserstoff, Acetyl, C₁-C₄-Alkyl, C₁-C₄-Hydroxyalkyl, C₁-C₄-Aminoalkyl bedeutet,
wobei alle Alkyl- oder Alkoxyreste der oben definierten Gruppen geradkettig und verzweigt sein können,
und mit der Maßgabe, daß
(i) A verschieden von 4-Dimethylaminophenyl und 4-Diethylaminophenyl ist, wenn R₁ Phenyl, R₂ Methyl, R₃ Wasserstoff darstellt, und
(ii) A verschieden von 2-Amino-4-hydroxy-5-methylphenyl und 2,4-Diaminophenyl ist, wenn R₁, R₂ und R₃ Wasserstoff bedeuten.

2. Zusammensetzung nach Anspruch 1, dadurch gekennzeichnet, daß sie eine Verbindung enthält, die ausgewählt ist unter:
- 5-Imino-4-[2'-amino-4'-hydroxy-5'-methylphenyl]-imino-1,3-dimethylpyrazolin,
- 5-Imino-4-[2'-amino-4'-hydroxy-5'-methylphenyl]-imino-1-methylpyrazolin,
- 5-Imino-4-[2'-amino-4'-hydroxy-5'-methylphenyl]-imino-3-methylpyrazolin,
- 5-Imino-4-[2'-amino-4'-hydroxyphenyl]-imino-1,3-dimethylpyrazolin,
- 5-Imino-4-[2'-amino-4'-hydroxyphenyl]-imino-1-methyl-pyrazolin,
- 5-Imino-4-[2'-amino-4'-hydroxyphenyl]-iminopyrazolin,
- 5-Imino-4-[2'-amino-4'-hydroxyphenyl]-imino-3-methyl-pyrazolin,
- 1-Ethyl-5-imino-4-[2'-amino-4'-hydroxy-5'-methylphenyl]-imino-3-methylpyrazolin,
- 1-Ethyl-5-imino-4-[2'-amino-4'-hydroxy-5'-methylphenyl]-iminopyrazolin,
- 1-Ethyl-5-imino-4-[2'-amino-4-hydroxy)-phenyl]-imino-3-methylpyrazolin,
- 1-Ethyl-5-imino-4-[2'-amino-4'-hydroxyphenyl]-iminopyrazolin,
- 1-Isopropyl-5-imino-4-[2'-amino-4'-hydroxy-5'-methylphenyl]-iminopyrazolin,
- 1-Isopropyl-5-imino-4-[2'-amino-4'-hydroxy-5'-methylphenyl]-imino-3-methylpyrazolin,
- 1-Isopropyl-5-imino-4-[2'-amino-4'-hydroxyphenyl]-iminopyrazolin,
- 1-Isopropyl-5-imino-4-[2'-amino-4'-hydroxyphenyl]-imino-3-methylpyrazolin,
- 1-β-Hydroxyethyl)-5-imino-4-[2'-amino-4'-hydroxyphenyl]-iminopyrazolin,
- 1-β-Hydroxyethyl)-5-imino-4-[2'-amino-4'-hydroxyphenyl]-imino-3-methylpyrazolin,
- 1-(β-Hydroxyethyl)-5-imino-4-[2'-amino-4'-hydroxy-5'-methylphenyl]-iminopyrazolin,
- 1-(β-Hydroxyethyl)-5-imino-4-[2'-amino-4'-hydroxy-5'-methylphenyl]-imino-3-methylpyrazolin,
- 5-Imino-4-[2'-amino-4'-hydroxy-5'-methylphenyl]-imino-3-trifluormethylpyrazolin,
- 1-Methyl-5-imino-4-[2'-amino-4'-hydroxy-5'-methylphenyl]-imino-3-trifluormethylpyrazolin,
- 1-Methyl-5-imino-4-[2'-amino-4'-hydroxyphenyl]-imino-3-trifluormethylpyrazolin,
- 5-Imino-4-[2'-amino-4'-hydroxyphenyl]-imino-3-trifluormethylpyrazolin,
- 1-Ethyl-5-imino-4-[2'-amino-4'-hydroxy-5'-methylphenyl]-imino-3-trifluormethylpyrazolin,
- 1-Ethyl-5-imino-4-[2'-amino-4'-hydroxyphenyl]-imino-3-trifluormethylpyrazolin,
- 1-Isopropyl-5-imino-4-[2'-amino-4'-hydroxy-5'-methylphenyl]-imino-3-trifluormethylpyrazolin,
- 1-Isopropyl-5-imino-4-[2'-amino-4'-hydroxyphenyl]-imino-3-trifluormethylpyrazolin,
- 1-(β-Hydroxyethyl)-5-imino-4-[2'-amino-4'-hydroxyphenyl]-imino-3-trifluormethylpyrazolin,
- 1-(β-Hydroxyethyl)-5-imino-4-[2'-amino-4'-hydroxy-5'-methylphenyl]-imino-3-trifluormethylpyrazolin,
und/oder ihre entsprechende tautomere 4-Ylidenamino-5-amino-pyrazol-Form, wenn die Tautomerie möglich ist.

3. Zusammensetzung nach einem der vorhergehenden Ansprüche, dadurch gekennzeichnet, daß sie einen pH-Wert im Bereich von 4 bis 11 aufweist.

4. Verwendung der Verbindungen der Formel (I) oder (II), die wie in Anspruch 1 definiert sind, als direktziehender Farbstoff in oder für die Herstellung von Färbemittel zusammensetzungen für Keratinfasern.

5. Verwendung der Verbindungen der Formel (I) oder (II), die wie in Anspruch 1 definiert sind, als direktziehender Farbstoff in oder für die Herstellung von Zusammensetzungen, die für die aufhellende direkte Färbung von Keratinfasern vorgesehen sind.

6. Zusammensetzung zur direkten Färbung von Keratinfasern, insbesondere von menschlichen Keratinfasern wie den Haaren, dadurch gekennzeichnet, daß sie in einem für die Färbung geeigneten Medium mindestens einen direktziehenden Farbstoff der Formel (I) oder (II) enthält, der wie in Anspruch 1 oder 2 definiert ist.

7. Zusammensetzung zur aufhellenden direkten Färbung von Keratinfasern, insbesondere von menschlichen Keratinfasern wie den Haaren, dadurch gekennzeichnet, daß sie in einem für die Färbung geeigneten Medium mindestens einen direktziehenden Farbstoff der Formel (I) oder (II) enthält, der wie in Anspruch 1 oder 2 definiert ist.

8. Gebrauchsfertige Zusammensetzung nach Anspruch 7, dadurch gekennzeichnet, daß sie außerdem ein Oxidationsmittel, ein Alkalisierungsmittel in einer Menge, die ausreicht, um den pH-Wert der fertigen Zusammensetzung auf einen Wert oberhalb 7 einzustellen, enthält.

9. Zusammensetzung nach einem der vorhergehenden Ansprüche, dadurch gekennzeichnet, daß die Verbindung(en) der Formel (I) oder (II) in einer Konzentration von 0,001 bis 5 Gew.-%, bezogen auf das Gesamtgewicht der Zusammensetzung vor dem Auftragen auf die Fasern enthalten ist/sind.

10. Zusammensetzung nach einem der vorhergehenden Ansprüche, dadurch gekennzeichnet, daß die Verbindung(en) der Formel (I) oder (II) in einer Konzentration von 0,01 bis 3 Gew.-%, bezogen auf das Gesamtgewicht der Zusammensetzung vor dem Auftragen auf die Fasern enthalten ist/sind.

11. Zusammensetzung nach Anspruch 8, dadurch gekennzeichnet daß das Oxidationsmittel aus Wasserstoffperoxid besteht.

12. Zusammensetzung nach Anspruch 8, dadurch gekennzeichnet, daß das Alkalisierungsmittel unter Ammoniak und Alkanolaminen ausgewählt ist.

13. Zusammensetzung nach einem der Ansprüche 8 bis 12, dadurch gekennzeichnet, daß das Alkalisierungsmittel in einer Menge enthalten ist, die ausreicht, um den pH-Wert der fertigen Zusammensetzung auf einen Wert im Bereich von 8,5 bis 11 einzustellen.

14. Zusammensetzung nach einem der vorhergehenden Ansprüche, dadurch gekennzeichnet, daß sie andere direktziehende Farbstoffe enthält, die unter Nitrobenzol-Farbstoffen wie Nitrophenylendiaminen, Nitrodiphenylaminen, Nitroanilinen, Nitrophenolethern und Nitrophenolen, Nitropyridinen, Anthrachinon-, Mono- oder Diazo-, Triarylmethan-, Azin-, Acridin, Xanthen-Farbstoffen oder metallhaltigen Farbstoffen ausgewählt werden.

15. Zusammensetzung nach einem der vorhergehenden Ansprüche, dadurch gekennzeichnet, daß das für die Färbung geeignete Medium ein wäßriges Medium ist, das aus Wasser und/oder organischen Lösungsmittel, die unter Alkoholen, Glykolen und Glykolethern ausgewählt ist, in Anteilen von 0,5 bis 20 Gew.-%, bezogen auf das Gesamtgewicht der Zusammensetzung, besteht.

16. Verfahren zur Färbung von Keratinfasern und insbesondere von menschlichen Keratinfasern wie den Haaren durch direkte Färbung, dadurch gekennzeichnet, daß die wie in den Ansprüchen 1 bis 3, 6, 9, 10, 14 und 15 definierte Färbemittelzusammensetzung auf die trockenen oder feuchten Keratinfasern aufgetragen wird und die Fasern ohne zwischenzeitliches Spülen getrocknet werden.

17. Verfahren zur Färbung von Keratinfasern und insbesondere von menschlichen Keratinfasern wie den Haaren durch direkte Färbung, dadurch gekennzeichnet, daß die wie in einem der Ansprüche 1 bis 3, 6, 9, 10, 14 und 15 definierte Färbemittelzusammensetzung auf die trockenen oder feuchten Keratinfasern aufgetragen wird und daß nach einer Einwirkzeit von etwa 3 bis 60 min die Fasern gespült, gegebenenfalls gewaschen, erneut gespült und anschließend getrocknet werden.

18. Verfahren zur Färbung von Keratinfasern und insbesondere von menschlichen Keratinfasern wie den Haaren durch aufhellende direkte Färbung, dadurch gekennzeichnet, daß die wie in einem der Ansprüche 1, 2 und 7 bis 15 definierte Färbemittelzusammensetzung (A) aufgetragen wird, wobei eine Aufhellung der Fasern bei einem pH-Wert oberhalb 7 mit Hilfe eines Oxidationsmittels hervorgerufen wird, das im Augenblick der Verwendung zu der Färbemittelzusammensetzung gegeben wird oder das in einer gleichzeitig angewendeten Zusammensetzung (B) enthalten ist.

19. Vorrichtungen mit mehreren Abteilungen oder "Kits" für die aufhellende Färbung von Keratinfasern wie den Haaren, dadurch gekennzeichnet, daß sie mindestens zwei Abteilungen enthalten, von denen eine Abteilung eine wie in einem der Ansprüche 1, 2 und 7 bis 15 definierte Zusammensetzung (A) und eine weitere Abteilung eine Zusammensetzung (B) enthält, die in einem für die Färbung geeigneten Medium ein Oxidationsmittel enthält.

20. 4,5-Diiminopyrazoline der wie in Anspruch 1 definierten Formeln (I) oder (II) mit der Maßgabe, daß
(i) A verschieden von 4-Dimethylaminophenyl und 4-Diethylaminophenyl ist, wenn R₁ Phenyl, R₂ Methyl, R₃ Wasserstoff darstellt, und
(ii) A verschieden von p-(2-Methyl-5-aminophenol) und p-(1,3-Diaminobenzol) ist, wenn R₁, R₂ und R₃ Wasserstoff bedeuten.

21. Verfahren zur Herstellung einer wie in Anspruch 20 definierten Verbindung der Formel (II), dadurch gekennzeichnet, daß es darin besteht, in einem wäßrig-alkoholischen Medium bei einer Temperatur von 10 bis 60 °C ein 4,5-Diaminopyrazol der Formel (3) 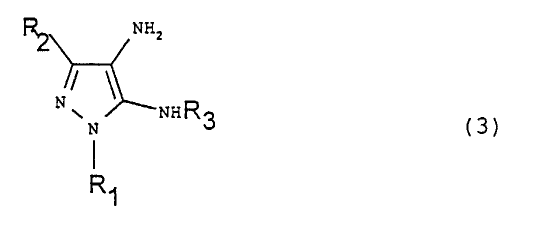 mit einer Verbindung der Formel (4) 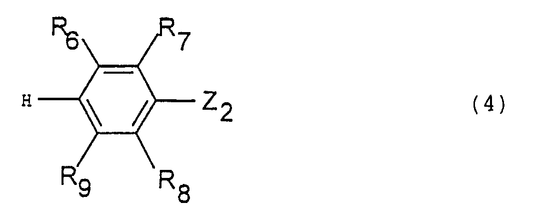 umzusetzen.

22. Verfahren nach Anspruch 21, dadurch gekennzeichnet, daß die Verbindung der Formel (4) eine Verbindung der Formel 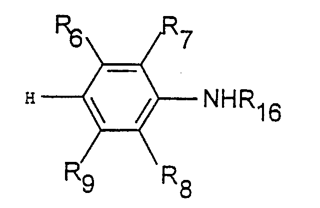 ist, in der R₆, R₇, R₈, R₉ dasselbe wie die Reste R₆ bis R₉ bedeuten, die weiter oben in Anspruch 20 angegeben wurden, und R₁₆ Wasserstoff oder geradkettiges oder verzweigtes C₁-C₄-Alkyl darstellt.

23. Verfahren nach Anspruch 21, dadurch gekennzeichnet, daß die Verbindung der Formel (4) eine Verbindung der Formel 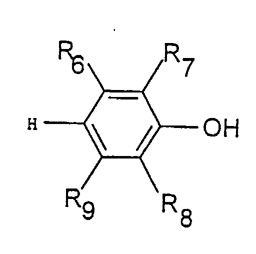 ist.

24. 5-Amino-2-methyl-N-(5-amino-1-methylpyrazol-4-yl)-p-chinonmonoimin sowie seine tautomere Form 5-Imino-4-[2-amino-4'-hydroxy-5'-methyl)-phenyl]-imino-1-methylpyrazolin nach Anspruch 20.

25. 5-Amino-2-methyl-5-(amino-1,3-dimethylpyrazol-4-yl)-p-chinonmonoimin sowie seine tautomere Form 5-Imino-4-[2'-amino-4'-hydroxy-5'-methyl)-phenyl]-imino-1,3-dimethylpyrazolin nach Anspruch 20.

## Claims

1. Dye composition for keratin fibres, in particular for human keratin fibres such as the hair, characterized in that it contains, in a medium which is suitable for dyeing, at least one 4,5-diiminopyrazoline compound of formula (I): 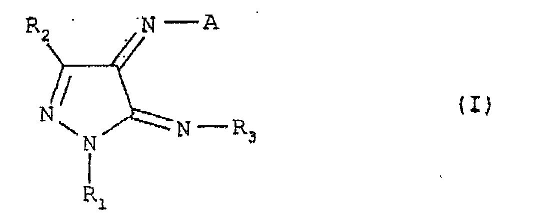 and/or its 4-ylideneamino-5-aminopyrazole tautomeric form of formula (II): 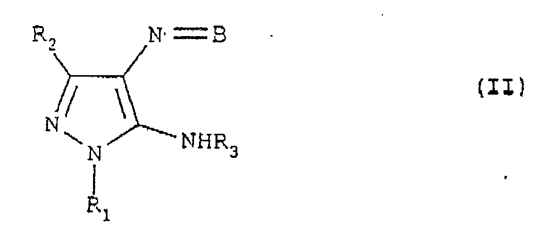 in which,
- R₁ and R₃, which may be identical or different, represent a hydrogen atom, a C₁-C₆ alkyl radical, a C₂-C₄ hydroxyalkyl radical or a C₂-C₄ aminoalkyl radical, a phenyl radical or a phenyl radical substituted with a halogen atom or with a C₁-C₄ alkyl, C₁-C₄ alkoxy, nitro, trifluoromethyl, amino or C₁-C₄ alkylamino radical, a benzyl radical or a benzyl radical substituted with a halogen atom or with a C₁-C₄ alkyl, C₁-C₄ alkoxy, methylenedioxy or amino radical,
a radical: 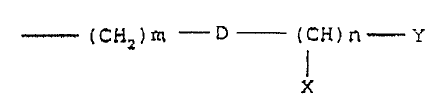 in which m and n denote an integer ranging from 1 to 3 inclusive,
D denotes oxygen or -NH-,
X denotes hydrogen or CH₃,
Y denotes CH₃ or OR₄, R₄ denoting hydrogen, methyl or ethyl,
and R₃ may also denote an amino radical,
- R₂ represents a hydrogen atom, a C₁-C₆ alkyl radical, a C₁-C₄ hydroxyalkyl radical, a C₁-C₄ aminoalkyl radical, a C₁-C₂ alkylthio radical, a C₁-C₅ alkoxy radical, a trifluoromethyl, cyano or carboxyl radical, a phenyl radical or a phenyl radical substituted with a halogen atom or with a C₁-C₄ alkyl, C₁-C₄ alkoxy, nitro, trifluoromethyl, amino or C₁-C₄ alkylamino radical, a benzyl radical or a benzyl radical substituted with a halogen atom or with a C₁-C₄ alkyl, C₁-C₄ alkoxy, nitro, trifluoromethyl, amino or C₁-C₄ alkylamino radical, a heterocycle chosen from thiophene, furan or pyridine,
a radical:
―(CH₂)p―O―(CH₂)q―OR₅
in which p and q, which may be identical or different, are integers ranging from 1 to 3 inclusive, and R₅ denotes hydrogen or methyl,
A represents, in formula (I), the radical: 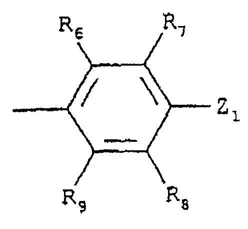 and
- B represents, in formula (II), the radical: 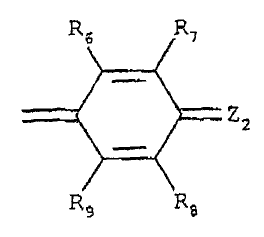 in which radicals,
Z₁ represents a hydroxyl radical, a C₁-C₄ alkoxy radical, an amino radical, an acetamido radical, a C₁-C₄ alkylamino radical, a C₁-C₄ hydroxyalkylamino radical, a C₁-C₄ amino-alkylamino radical, a radical NR₁₀R₁₁ in which R₁₀ and R₁₁, which may be identical or different, denote a C₁-C₄ alkyl, C₂-C₄ hydroxyalkyl or C₃-C₄ aminoalkyl radical,
R₆, R₇, R₈ and R₉, which may be identical or different, represent a hydrogen atom, a C₁-C₄ alkyl radical, a C₁-C₄ alkoxy radical, a C₁-C₄ alkylthio radical, an amino radical, a C₁-C₄ alkylamino radical, a carbamoylmethylamino radical, a C₂-C₄ hydroxyalkyl radical, a C₂-C₄ monohydroxyalkoxy radical, a C₂-C₄ dihydroxyalkoxy radical, a C₁-C₂ carboxyalkoxy radical, a chlorine, bromine or fluorine atom, a C₁-C₂ trifluoroalkyl radical,
a radical: 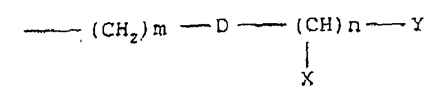 in which m, n, D, X and Y have the same meanings as above, or a radical NR₁₀R₁₁ where R₁₀ and R₁₁ have the same meanings as above,
Z₂ represents O or NR with R denoting H, acetyl, C₁-C₄ alkyl, C₁-C₄ hydroxyalkyl or C₁-C₄ aminoalkyl,
it being understood that all the alkyl or alkoxy radicals of the groups defined above may be linear or branched, and with the proviso that,
(i) when R₁ denotes phenyl, R₂ denotes methyl and R₃ denotes hydrogen, then A is other than 4-dimethylaminophenyl or 4-diethylaminophenyl,
(ii) when R₁, R₂ and R₃ denote hydrogen, then A is other than the 2 -amino-4-hydroxy-5-methylphenyl and 2,4-diaminophenyl radicals.

2. Composition according to Claim 1, characterized in that it contains a compound chosen from the group consisting of:
5-imino-4-[(2'-amino-4'-hydroxy-5'-methyl)phenyl]imino-1,3-dimethylpyrazoline,
5-imino-4-[(2'-amino-4'-hydroxy-5'-methyl)phenyl]imino-1-methylpyrazoline,
5-imino-4-(2'-amino-4'-hydroxy-5'-methyl)phenyl]imino-3-methylpyrazoline,
5-imino-4-[(2'-amino-4'-hydroxy)phenyl]imino-1,3-dimethylpyrazoline,
5-imino-4-[(2 '-amino-4'-hydroxy)phenyl]imino-1-methylpyrazoline,
5-imino-4-[(2'-amino-4'-hydroxy)phenyl]iminopyrazoline,
5-imino-4-[(2'-amino-4'-hydroxy)phenyl]imino-3-methylpyrazoline,
1-ethyl-5-imino-4-[(2'-amino-4'-hydroxy-5'-methyl)-phenyl]imino-3-methylpyrazoline,
1-ethyl-5-imino-4-[(2'-amino-4'-hydroxy-5'-methyl)-phenyl]iminopyrazoline,
1-ethyl-5-imino-4-[(2'-amino-4'-hydroxy)phenyl]imino-3-methylpyrazoline,
1-ethyl-5-imino-4-[(2'-amino-4'-hydroxy)-phenyl]iminopyrazoline,
1-isopropyl-5-imino-4-[(2'-amino-4'-hydroxy-5'-methyl)phenyl]iminopyrazoline,
1-isopropyl-5-imino-4-[(2'-amino-4'-hydroxy-5'-methyl)phenyl]imino-3-methylpyrazoline,
1-isopropyl-5-imino-4-[(2'-amino-4'-hydroxy)phenyl]iminopyrazoline,
1-isopropyl-5-imino-4-[(2'-amino-4'-hydroxy)phenyl]imino-3-methylpyrazoline,
1-(β-hydroxyethyl)-5-imino-4-[(2'-amino-4'-hydroxy)phenyl]iminopyrazoline,
1-(β-hydroxyethyl)-5-imino-4-[(2'-amino-4'-hydroxy)phenyl]imino-3-methylpyrazoline,
1-(β-hydroxyethyl)-5-imino-4-[(2'-amino-4'-hydroxy-5'-methyl)phenyl]iminopyrazoline,
1-(β-hydroxyethyl)-5-imino-4-[(2'-amino-4'-hydroxy-5'-methyl)phenyl]imino-3-methylpyrazoline,
5-imino-4-[(2'-amino-4'-hydroxy-5'-methyl)phenyl]imino-3-trifluoromethylpyrazoline,
1-methyl-5-imino-4-[(2'-amino-4'-hydroxy-5'-methyl)phenyl]imino-3-trifluoromethylpyrazoline,
1-methyl-5-imino-4-[(2'-amino-4'-hydroxy)phenyl]imino-3-trifluoromethylpyrazoline,
5-imino-4-[(2'-amino-4'-hydroxy)phenyl]imino-3-trifluoromethylpyrazoline,
1-ethyl-5-imino-4-[(2'-amino-4'-hydroxy-5'-methyl)phenyl]imino-3-trifluoromethylpyrazoline,
1-ethyl-5-imino-4-[(2'-amino-4'-hydroxy)phenyl]imino-3-trifluoromethylpyrazoline,
1-isopropyl-5-imino-4-[(2'-amino-4'-hydroxy-5'-methyl)phenyl]imino-3-trifluoromethylpyrazoline,
1-isopropyl-5-imino-4-[(2'-amino-4'-hydroxy)phenyl]imino-3-trifluoromethylpyrazoline,
1-(β-hydroxyethyl)-5-imino-4-[(2'-amino-4'-hydroxy)phenyl]imino-3-trifluoromethylpyrazoline,
1-(β-hydroxyethyl)-5-imino-4-[(2'-amino-4'-hydroxy-5'-methyl)phenyl]imino-3-trifluoromethylpyrazoline, and/or their corresponding 4-ylideneamino-5-aminopyrazole tautomeric form, when tautomerism is possible.

3. Composition according to either of the preceding claims, characterized in that it has a pH of between 4 and 11.

4. Use of the compounds of formula (I) or (II) as defined in Claim 1, as direct dye in, or for the preparation of, compositions for dyeing keratin fibres.

5. Use of the compounds of formula (I) or (II) as defined in Claim 1, as direct dye in, or for the preparation of, compositions intended for the lightening direct dyeing of keratin fibres.

6. Direct dye composition for keratin fibres, in particular for human keratin fibres such as the hair, characterized in that it contains, in a medium which is suitable for dyeing, at least one direct dye of formula (I) or (II) as defined in Claim 1 or 2.

7. Lightening direct dye composition for keratin fibres, in particular for human keratin fibres such as the hair, characterized in that it contains, in a medium which is suitable for dyeing, at least one direct dye of formula (I) or (II) as defined in Claim 1 or 2.

8. Ready-to-use composition according to Claim 7, characterized in that it also contains an oxidizing agent and a basifying agent in an amount which is sufficient to adjust the final pH to a value above 7.

9. Composition according to any one of the preceding claims, characterized in that the compound or compounds of formula (I) or (II) are present in a concentration ranging from 0.001 to 5 % by weight relative to the total weight of the composition before they are applied to the fibres.

10. Composition according to any one of the preceding claims, characterized in that the compound or compounds of formula (I) or (II) are present in a concentration ranging from 0.01 to 3 % by weight relative to the total weight of the composition before they are applied to the fibres.

11. Composition according to Claim 8, characterized in that the oxidizing agent consists of aqueous hydrogen peroxide solution.

12. Composition according to Claim 8, characterized in that the basifying agent is chosen from aqueous ammonia and an alkanolamine.

13. Composition according to any one of Claims 8 to 12, characterized in that the basifying agent is present in an amount which is sufficient to adjust the final pH to between values ranging from 8.5 to 11.

14. Composition according to any one of the preceding claims, characterized in that it contains other direct dyes chosen from nitrobenzene dyes, for instance nitrophenylenediamines, nitrodiphenylamines, nitroanilines, nitrophenyl ethers or nitrophenols, nitropyridines, anthraquinone, monoazo or diazo, triarylmethane, azine, acridine and xanthene dyes, or metalliferous dyes.

15. Composition according to any one of the preceding claims, characterized in that the medium which is suitable for dyeing is an aqueous medium consisting of water and/or organic solvents chosen from alcohols, glycols and glycol ethers, in proportions of between 0.5 and 20 % by weight relative to the total weight of the composition.

16. Process for dyeing keratin fibres, and in particular human keratin fibres such as the hair, by direct dyeing, characterized in that the dye composition defined according to Claims 1 to 3, 6, 9, 10, 14 and 15 is applied to the wet or dry keratin fibres, and these fibres are dried without intermediate rinsing.

17. Process for dyeing keratin fibres, and in particular human keratin fibres such as the hair, by direct dyeing, characterized in that the dye composition defined according to any one of Claims 1 to 3, 6, 9, 10, 14 and 15 is applied to the wet or dry keratin fibres and, after the composition has been left to stand on the fibres for 3 to 60 minutes approximately, the fibres are rinsed, optionally washed, rinsed again and then dried.

18. Process for dyeing keratin fibres, and in particular human keratin fibres such as the hair, by lightening direct dyeing, characterized in that the dye composition (A) defined according to any one of Claims 1, 2 and 7 to 15 is applied by lightening the said fibres at a pH above 7 using an oxidizing agent which is added to the said dye composition only at the time of use, or which is present in a composition (B) that is applied simultaneously.

19. Multi-compartment devices or "kits" for the lightening dyeing of human keratin fibres such as the hair, characterized in that they contain at least two compartments, one of which contains composition (A) as defined in any one of Claims 1, 2 and 7 to 15, and another of which contains a composition (B) comprising an oxidizing agent in a medium which is suitable for dyeing.

20. 4,5-Diiminopyrazolines of formulae (I) or (II) defined according to Claim 1, with the proviso that,
(i) when R₁ denotes phenyl, R'₂ denotes methyl and R'₃ denotes hydrogen, then A is other than 4-dimethylaminophenyl and 4-diethylaminophenyl,
(ii) when R₁, R₂ and R₃ denote hydrogen, then A is other than the 2-amino-4-hydroxy-5-methylphenyl and 2,4-diaminophenyl radicals.

21. Process for the preparation of a compound of formula (II) as defined in Claim 20, characterized in that it consists in reacting, in aqueous-alcoholic medium and at a temperature of between 10 and 60°C, a 4,5-diaminopyrazole of formula (3): 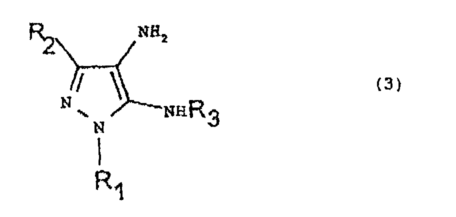 with a compound of formula (4):

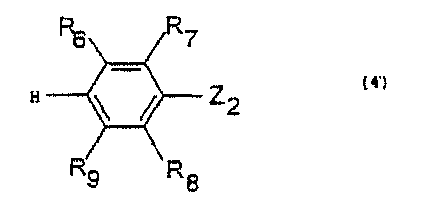

22. Process according to Claim 21, characterized in that the compound of formula (4) is a compound of formula: 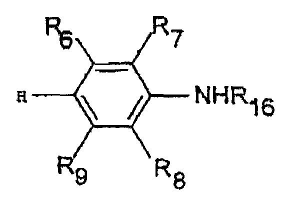 in which
R₆, R₇, R₈ and R₉ have the same meanings as the radicals R₆ to R₉ denoted above in Claim 20, and R₁₆ denotes hydrogen or linear or branched C₁-C₄ alkyl.

23. Process according to Claim 21, characterized in that the compound of formula (4) is a compound of formula:

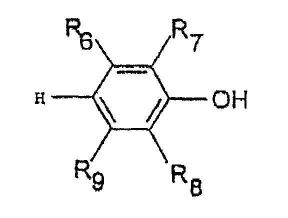

24. 5-Amino-2-methyl-N-(5-amino-1-methylpyrazol-4-yl)-p-quinone monoimine and its 5-imino-4-[(2'-amino-4'-hydroxy-5'-methyl)phenyl]imino-1-methylpyrazoline tautomeric form, according to Claim 20.

25. 5-Amino-2-methyl-N-(5-amino-1,3-dimethylpyrazol-4-yl)-p-quinone monoimine and its 5-imino-4-[(2'-amino-4'-hydroxy-5'-methyl)phenyl]imino-1,3-dimethylpyrazoline tautomeric form, according to Claim 20.
